# EUROPEAN PATENT APPLICATION

(11) **EP 4 406 536 A2**
(43) Date of publication of application: **31.07.2024**
(21) Application number: 24168742.5
(22) Date of filing: 29.01.2014
(51) Int. Cl.: A61K 31/198

(54) **CYSTATHIONINE BETA-SYNTHASE ENZYME FOR TREATMENT OF HOMOCYSTINURIA**

(30) Priority: 29.01.2013 US 201361758138 P; 14.03.2013 US 201313803804
(62) Divisional of application: 20168207.7
(71) Applicant: The Regents of The University of Colorado, A Body Corporate, Denver, CO 80203 (US)
(72) Inventor: Kraus, Jan P., Littleton, CO 80123 (US); Majtan, Tomas, Aurora, CO 80011 (US); Bublil, Erez, 4481600 Ets Efraim (IL)
(74) Representative: Hoffmann Eitle

(57) **Abstract**

The invention provides reagents and methods for enzyme replacement therapy using human cystathionine β-synthase (CBS) homologues, variants of mutants thereof to treat homocystinuria and other related diseases and disorders.

## Description

### CROSS-REFERENCE TO RELATED APPLICATIONS

This application claims the benefit of U.S. Provisional Application No: 61/758,138, filed January 29, 2013, and U.S. non-provisional Application No: 13/803,804, filed March 14. 2013, the disclosures of each of which are incorporated by reference.

### FIELD OF THE INVENTION

The present invention generally relates to compositions suitable for enzyme replacement therapy comprising forms of human Cystathionine Beta-Synthase (CBS) which can significantly reduce serum homocysteine (Hey) concentrations and increase the production of the downstream metabolites such as cystathionine and cysteine. Such compositions can be used for treatment of conditions or diseases such as homocystinuria and homocysteine remethylation disorders.

### BACKGROUND OF THE INVENTION

CBS, a central enzyme in the transsulfuration pathway, plays an essential role in homocysteine (Hey) metabolism in eukaryotes (Mudd et al., 2001, in THE METABOLIC AND MOLECULAR BASES OF INHERITED DISEASE, 8 Ed., pp. 2007-2056, McGraw-Hill, New w York). The CBS enzyme catalyzes a pyridoxal-5'-phosphate (PLP; Vitamin B₆)-dependent condensation of serine and homocysteine to form cystathionine, which is then used to produce cysteine by another PLP-dependent enzyme, cystathionine γ-lyase. When CBS activity is dramatically reduced or absent, as a result of certain genetic mutations, Hey builds up in tissues and blood. In mammalian cells that possess the transsulforation pathway, CBS occupies a key regulatory position between the re-methylation of Hey to methionine or its alternative use in the biosynthesis of cysteine.

In healthy normal individuals, CBS-mediated conversion of Hey to cystathionine is the rate-limiting intermediate step of methionine (Met) metabolism to cysteine (Cys). Vitamin B₆ is an essential coenzyme for this process. In individuals with certain genetic mutations in the CBS enzyme, the conversion of Hey to cystathionine is slowed or absent, resulting in elevations in the serum concentrations of the enzymatic substrate (Hey) and a corresponding decrease in the serum concentrations of the enzymatic product (cystathionine; Cth). The clinical condition of an elevated serum level of Hey, and its concomitant excretion into the urine, is collectively known as homocystinuria.

The estimates on the prevalence of homocystinuria vary widely. Data from newborn screening and clinical ascertainment provide a range of 1:200,000 to 1:335.000 live births (Mudd *et al.*, 2001). Recent evidence from DNA screening studies of newborns in Denmark, Germany, Norway and the Czech Republic found that the true incidence may be as high as ∼1:6,000 (Gaustadnes et al., 1999, N Engl J Med. 1340:1513: Linnebank et al., 2001, Thromb Haemost. 85:986; Refsum et al., 2004, Clin. Chem. 50:3; Sokolova et al., 2001, Hum Mutat.18:548). Additionally, recent work has indicated that CBS-deficient homocystinuria (CBSDH) individuals exist with either psychiatric or cardiovascular complications but are currently undiagnosed due to a lack of the characteristic connective tissue defects that are typically instrumental in diagnosis (Li and Stewart, 1999, Pathol. 31:221; Linnebank et al., 2003, J. Inherited Metabol. Dis. 26: 509; Maclean et al., 2002, Hum Mutat. 19:641). The primary health problems associated with CBSDH include: cardiovascular disease with a predisposition to thrombosis, resulting in a high rate of mortality in untreated and partially treated individuals; connective tissue problems affecting the ocular system with progressive myopia and lens dislocation; connective tissue problems affecting the skeleton characterized by marfanoid habitus, osteoporosis, and scoliosis; and central nervous system problems, including mental retardation and seizures.

The therapeutic resolution of CBS-associated homocystinuria is dependent upon the type of mutations present in the CBS gene. Approximately 160 pathogenic mutations of the CBS gene have been identified to date in humans. There is a functional trichotomy in the nature of pathogenic mutations associated with CBSDH. One group of mutations is classified as "pyridoxine-responsive," where CBS enzyme function can be restored by high dose Vitamin B₆ therapy. This treatment can be effective, but does not always mitigate the pathological events in these individuals, and some of the events occur even in these individuals over time. The second group of functional mutations is represented by the "C-terminal CBS mutants" that are defective in their ability to respond to post-translational up-regulation by S-adenosylmethionine. Individuals with this class of mutations usually lack the mental retardation and connective tissue aspects of the phenotype. This class is detected after measurement of plasma Hey levels following an idiopathic thrombotic event before the age of 40 years (Maclean et al., 2002, Hum Mutat.19: 641-55). The final group of CBSDH mutations is "classical homocystinuria," which represents the most severe form of the disease. For these latter two groups of individuals, Vitamin B₆ therapy in isolation does not effectively lower serum Hey levels.

The pathophysiology of homozygous CBS deficiency is undoubtedly complex, but there is a consensus that the fundamental instigator of end-organ injury is an extreme elevation of serum Hcy. The toxicity of profound elevations in blood and tissue concentrations of Hcy may ensue from the molecular reactivity and biological effects of Hey *per* se or from its metabolites (e.g., Hcy-thiolactone) that affect a number of biological processes (Jakubowski et al., 2008, FASEB J 22: 4071-6). Abnormalities in chronic platelet aggregation, changes in vascular parameters, and endothelial dysfunction have all been described in individuals with homocystinuria.

Currently, three treatment options exist for the treatment of CBSDH:
1) Increase of residual activity of CBS activity using pharmacologic doses of Vitamin B₆, in Vitamin B₆-responsive individuals;
2) Lowering of serum Hcy by a diet with a strict restriction of the intake of Met; and
3) Detoxification by betaine-mediated conversion of Hey into Met, thus lowering serum Hcy concentration.

Each of these three therapies is aimed at lowering serum Hey concentration. The standard treatment for individuals affected with Vitamin B₆ non-responsive CBSDH consists of a Met-restricted diet supplemented with a metabolic formula and Cys (which has become a conditionally essential amino acid in this condition). Intake of meat, dairy products and other food high in natural protein is prohibited. Daily consumption of a poorly palatable, synthetic metabolic formula containing amino acids and micronutrients is required to prevent secondary malnutrition. Supplementation with betaine (tradename: CYSTADANE^{™}, synonym: trimethylglycine) is also a standard therapy. Betaine serves as a methyl donor for the remethylation of Hcy to Met catalyzed by betaine-homocysteinemethyltransferase in the liver (Wilcken et al., 1983, N Engl J Med 309:, (8), 448-53). Dietary compliance generally has been poor, even in those medical centers where optimal care and resources are provided, and this non-compliance has major implications on the development of life-threatening complications of homocystinuria.

The evidence described in the above sections is summarized in the following points:
- Untreated homocystinuria has a high rate of complications in the vasculature, connective tissue, and central nervous system.
- Treatments that lower serum Hey, such as a severely Met-restricted diet and betaine, lower the associated clinical problems if executed well. Improvements in cognitive performance require treatment initiation in early infancy.

- Compliance with diet is uniformly poor. In individuals initiating therapy in the neonatal period, the loss of compliance occurs in adolescence. In individuals beginning therapy after the neonatal period, compliance is abysmal at all ages. The extreme difficulties of the current treatment approach are most readily evident in individuals who experience life-threatening symptoms preventable by diet, and yet are unable to adhere to this treatment.
- Failure of dietary compliance causes increased serum Hey, resurgence of complications in the vascular and connective tissue including fatal and incapacitating events, and risks severe side-effects such as cerebral edema (from excessive serum Met concentration) or severe malnutrition (from lack of essential amino acids).
- The most effective therapeutic strategy is to increase enzyme activity, as is evident when given pyridoxine to Vitamin B₆ responsive homocystinuria. This strategy is not possible for Vitamin B₆ non-responsive individuals due to the mutation status, and increased enzyme activity in these individuals will depend on the delivery of exogenous enzyme, *i.e*. enzyme replacement therapy (ERT) (a strategy that has never been attempted for treating homocystinuria).
- Of the three current treatment strategies with demonstrated efficacy: (1) increasing enzyme activity with pyridoxine in Vitamin B₆ responsive individuals; (2) reducing accumulating metabolites by a Met-restricted diet; and (3) detoxification by enzyme activity of the betaine-homocysteine methyltransferase in betaine treatment, all have in common a lowering of total Hey in plasma (Walter, et al., 1998, Eur J Pediatr 157(Supp12): S71-6).

In addition, for all current treatment strategies used in human patients (except B6 supplementation, which is appropriate for only a subset of homocystinurics), reduction in homocysteine is not accompanied by increases in Cth or Cys. Because it has not been established that excess Hey, rather than a deficiency of a downstream metabolite, is responsible for clinical symptoms, current therapies that are limited to reducing homocysteine may be inadequate for providing robust and effective treatment options.

Thus, there remains a need in the art for more effective treatment strategies for individuals with homocystinuria.

### SUMMARY OF THE INVENTION

As set forth herein, the invention provides compositions, specifically compositions, and methods for *inter alia* reducing serum Hcy in individuals with homocystinuria. Also provided are compositions, specifically pharmaceutical compositions, which restore substantially normal levels of metabolites including but not limited to methionine, such as cysteine and cystathionine. As described more specifically herein, reagents and methods for enzyme replacement therapy (ERT) for homocystinuria are provided wherein modified forms of the natural enzyme have been recombinantly engineered to improve its pharmaceutical acceptability by *inter alia* providing improved stability, activity, and medicinal utility *in* vivo.

In a first aspect, the invention provides an isolated CBS polypeptide comprising SEQ ID NO: 02, wherein the isolated CBS polypeptide comprises a chemical modification and is a genetically engineered truncation thereof of the full-length human CBS protein at the amino terminus, carboxyl terminus or both amino and carboxyl termini. In one embodiment, the invention provides an engineered variant of human CBS that constitutes recombinant human CBS wherein the C-terminal regulatory region has been removed (*for example*, rhCBSΔC - the rhCBSΔC has truncated 138 residues at the carboxyl terminus and comprises amino acids 1-413 of the 551 amino acids of the full length protein) (SEQ ID NO: 3). In some embodiments, the species of the rhCBSΔC are mutated, in a preferred embodiment the truncated mutant CBS is rhCBSAC-CISS (or as abbreviated "C15S" - the C15S has truncated 138 residues at the carboxyl terminus; same as rhCBSΔC) (SEQ ID NO: 13), wherein a cysteine residue in position 15 in the CBS amino acid sequence is changed to a serine. In the particular embodiments disclosed herein include species of the truncated recombinant human CBS that are chemically modified, particularly by covalent attachment of polyethyleneglycol (PEG) moieties of the C-terminally truncated recombinant human CBS, in particular rhCBSΔC. These PEGylated species will be referred to as PEG-rhCBSΔC or PEG-C15S, respectively. These compositions (modified recombinant enzymes) and methods of use, particularly therapeutic use as set forth herein, enable individuals with homocystinuria to enjoy a far less restrictive diet (*e.g*., daily intake of 2 g or more of protein per kg), and have significantly decreased Hey plasma levels and substantially normalized metabolite levels including but not limited to methionine, such as cysteine and cystathionine, leading in the long-term to clinical improvement.

The invention advantageously enables individuals to achieve good control of serum Hey levels without the use of an extremely restricted diet, which has an unacceptable noncompliance rate. Use of the said truncated species, particularly species that are mutated, in particular said C15S mutants, PEGylated or both mutated and PEGylated maybe advantageously accompanied by improved metabolism in an individual, and hence improved clinical outcomes as reflected in decreased incidence of morbidity and mortality, associated *inter alia* with significantly reduced serum Hey concentrations.

As disclosed herein, the C15S mutated human CBS species advantageously adopts a conformation having significantly enhanced medicinal utility *in vivo* (*e.g.,* no detectable levels of aggregation). The C15S mutated human CBS species also exhibits highly reproducible expression, purification, and PEGylation patterns, and the C15S mutation reduces formation of aggregates, thus improving yields by improving recovery, and enables more consistent and reproducible PEGylation. These aspects can also be useful for quality issues, which are particularly relevant for steps prior to *in vivo* usefulness.

As disclosed herein, the PEGylated species advantageously have an increased size compared with the non-PEGylated truncated variants, particularly said rhCBSΔC or C15S species, wherein there is reduced or delayed clearance of the PEGylated species. In addition, chemical modification with PEG masks potential immunogenic epitopes on the surface of a protein and hinders access to the protein for proteolytic enzymes. PEGylation also advantageously alters the physico-chemical properties of the rhCBSΔC protein, thus modifying its bio-distribution, stability, and solubility without significantly detracting from its potency.

These and other features and advantages of the present invention will be more fully understood from the following detailed description of the invention taken together with the accompanying claims. It is noted that the scope of the claims is defined by the recitations therein and not by the specific discussion of features and advantages set forth in the present description.

### BRIEF DESCRIPTION OF THE DRAWINGS OF THE INVENTION

The following detailed description of the embodiments of the present invention can be best understood when read in conjunction with the following drawings.

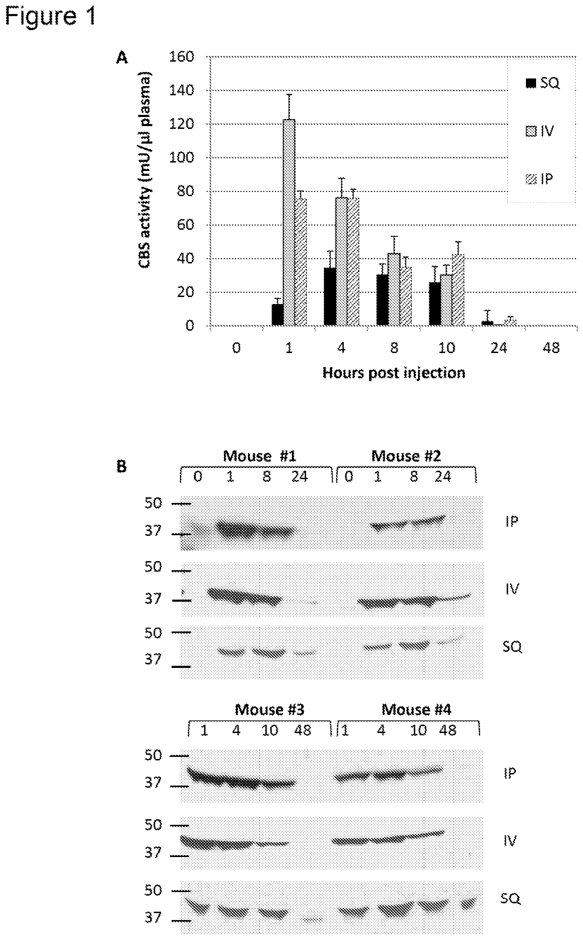

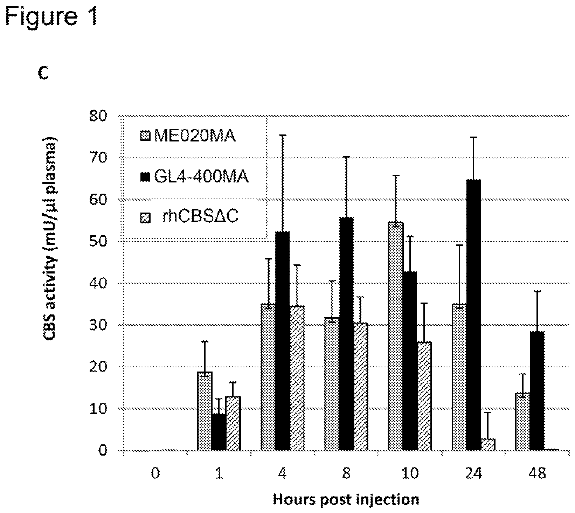

**Figure 1** illustrates experimental evidence for enhancement in plasma enzyme retention time *in vivo* following rhCBSΔC PEGylation. Figure 1 provides pharmacokinetic profiles after single administration of rhCBSΔC and PEGylated rhCBSΔC and evidence for their stability *in vivo*. **Figure la** is a bar graph showing the results of experiments wherein C57BL/6J mice were injected with 5 mg/kg body weight of the human truncated CBS (rhCBSΔC) *via* intra-peritoneal (IP), intra-vascular (IV) or sub-cutaneous (SQ) routes. Two experimental groups (denoted 1 and 2) of 5 mice each were used for each injection route (total n=30). For each injection route, blood was collected from group 1 at 0, 1, 8 and 24 hours post injection and from group 2 at 1, 4, 10, and 48 hours post injection. Plasma was analyzed for CBS activity using the radiometric activity assay (as described in Example 1). **Figure 1b** is a photograph of the results of Western blot analysis of CBS in plasma from two representative mice of each group as described in **Figure 1a** to follow CBS clearance from circulation. Following SDS-PAGE isolation of CBS on 12% gels and Western blotting to polyvinylidene difluoride (PVDF) membrane, the membranes were probed with rabbit polyclonal anti-hCBS antibody followed by secondary anti-rabbit antibody conjugated to horseradish peroxidase and the bands were visualized with SuperSignal West Pico Chemiluminescent Substrate (Pierce cat# 34077). **Figure 1c** is a bar graph showing the results of experiments where mice were injected with 5 mg/kg body weight of ME020MA- or GL4-400MA-PEGydated rhCBSΔC or with non-PEGylated rhCBSΔC via SQ route. ME020MA represents rhCBSΔC enzyme PEGylated with ME020MA, GL4-400MA represents rhCBSΔC enzyme PEGylated with GL4-400MA, and rhCBSΔC represents unmodified rhCBSΔC enzyme. Each treatment arm consisted of two groups of 5 mice which were bled, at the indicated time points, as described in Example 2 and shown in Figure 1A (total 30 mice). Plasma was analyzed for CBS activity using the radiometric activity assay.

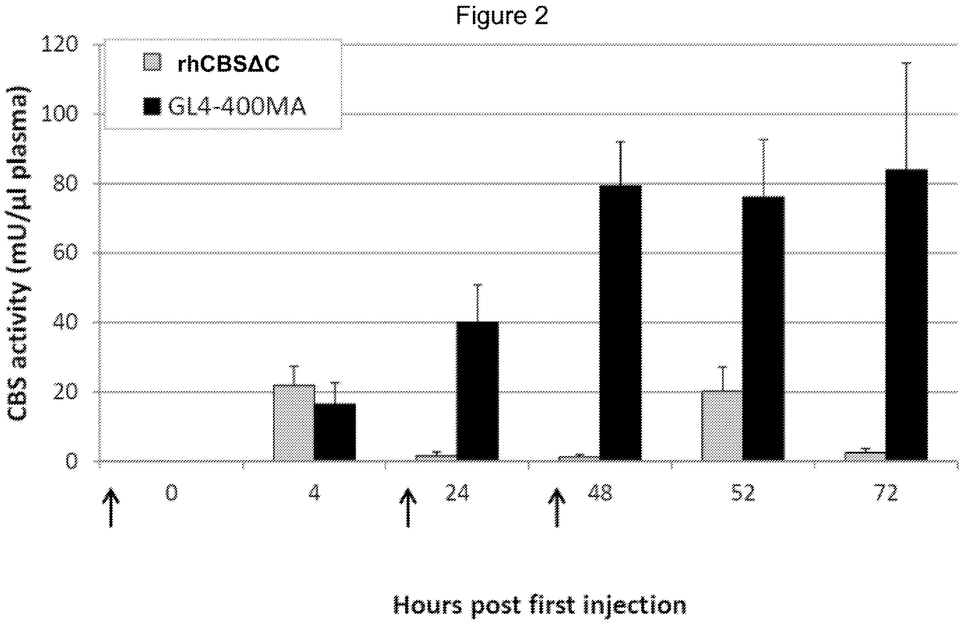

**Figure 2** is a bar graph showing that a stable level of CBS activity is achieved after repeated SQ administrations. Injection with the PEGylated, but not with the non-PEGylated rhCBSΔC, exhibits buildup of CBS activity *in vivo*. GL4-400MA represents rhCBSΔC enzyme PEGylated with GL4-400MA. and rhCBSΔC represents unmodified rhCBSΔC enzyme. C57BL/6J mice were injected at 0, 24, 48 hours (arrows) with 5 mg/kg body weight of non-PEGylated rhCBSΔC (n=5) or with GL4-400MA rhCBSΔC (n=5) and bled at the indicated time points. Plasma was analyzed for CBS activity using the radiometric activity assay described in Example 1c.

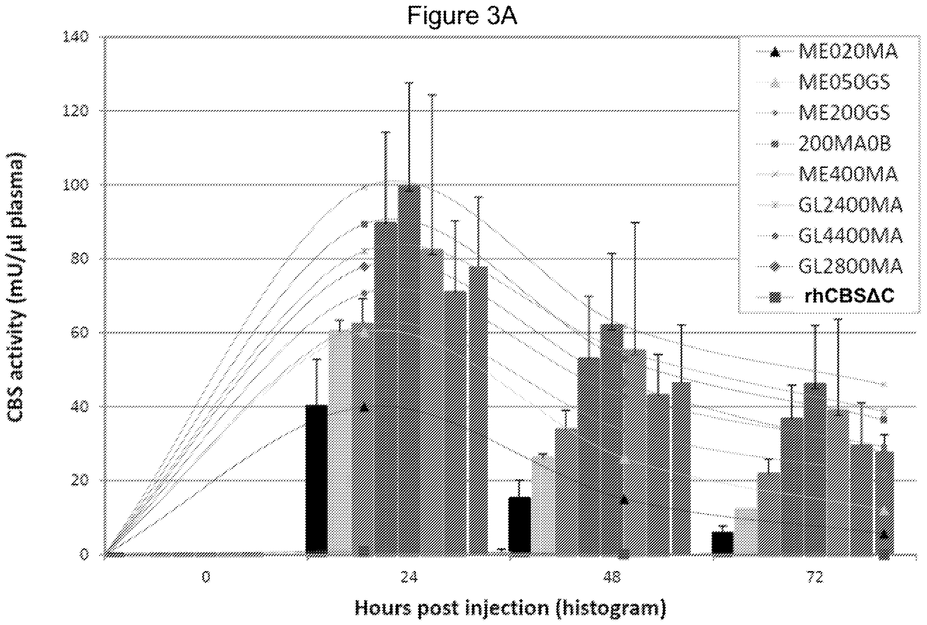

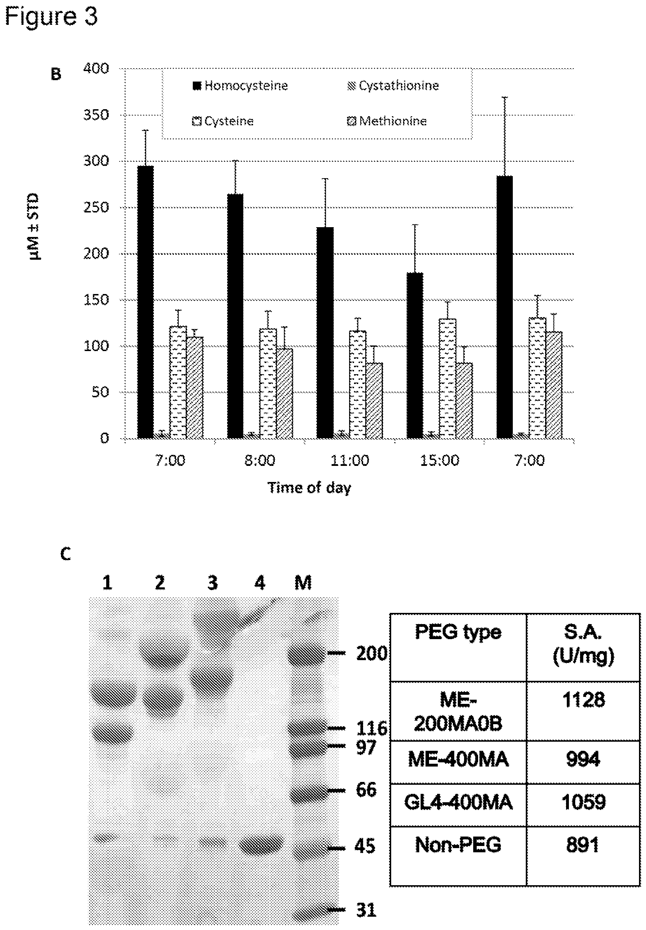

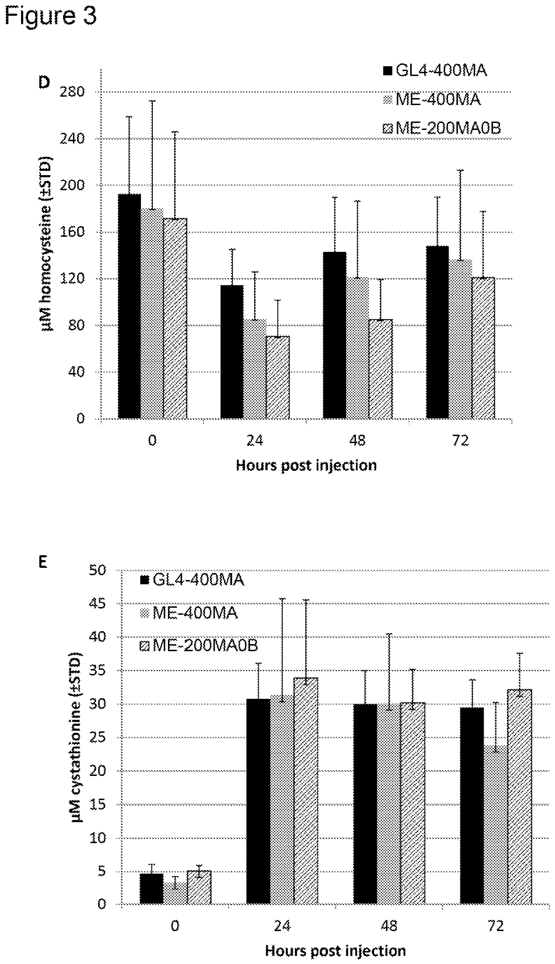

**Figure 3** illustrates experimental evidence that single injection of the PEGylated rhCBSΔC reduces homocysteine and increases cystathionine in plasma. **Figure 3a** is a graph showing that PEG modification of rhCBSΔC prolongs the systemic presence of CBS enzymatic activity following a single SQ administration of rhCBSΔC modified with the indicated types of activated PEG. The graph shows the results of experiments wherein twenty seven C57BL/6J mice were divided into 9 experimental groups (n=3). Each experimental group was injected via SQ route with 5 mg/kg body weight of rhCBSΔC PEGylated with the indicated PEG molecule, or injected with non-PEGylated enzyme. ME020MA represents rhCBSΔC enzyme PEGylated with ME020MA, ME050GS represents rhCBSΔC enzyme PEGylated with ME050GS, ME200GS represents rhCBSΔC enzyme PEGylated with ME200GS, 200MA0B represents rhCBSΔC enzyme PEGylated with 200MA0B, ME400MA represents rhCBSAC enzyme PEGylated with ME400MA, GL2400MA represents rhCBSΔC enzyme PEGylated with GL2400MA, GL4400MA represents rhCBSΔC enzyme PEGylated with GL4400MA, GL2800MA represents rhCBSΔC enzyme PEGylated with GL2800MA, and rhCBSΔC represents non-PEGylated rhCBSΔC enzyme. Blood samples were drawn at the indicated time points and CBS enzyme activity was determined using radiometric activity assay as described in Example 1e. Data is presented as a histogram with standard deviation (STD), and as a scatter plot. **Figure 3b** is a bar graph showing the natural diurnal variation in Hey, Cth, Cys, and Met levels in HO mouse plasma. The results are the average values of 6 HO mice (homocystinuric HO mouse model as described in Maclean et al., 2010, Mol. Genet. Metab. 101: 153-62) from which blood was drawn at the indicated time points throughout a 24-hour cycle. Plasma amino acid levels were determined by Stable-Isotope-Dilution Liquid Chromatography-Mass Spectrometry for each time point as described in Example 1e.
**Figure 3c** is a photograph of the results of electrophoretic analysis of rhCBSΔC PEGylated with ME-400MA (lane 1), GL4-400MA (lane 2) or ME-200MA0B (lane 3) resolved by electrophoresis along with non-PEGylated enzyme (lane 4), and stained with Coomassie blue (M indicates molecular weight standard). The specific activity (S.A.) for each of the PEGylated and non-PEGylated rhCBSΔC is indicated in the table.
**Figures 3d and 3e** are bar graphs showing the results of experiments wherein HO mice were injected via SQ route, once at time 0 with rhCBSΔC that had been PEGylated with the indicated PEG molecules, and bled at times 0 (prior to injection), 24, 48 and 72 hours post injection. Levels of plasma homocysteine (d) and cystathionine (e) for each of the groups (n=5-6) are indicated. For Figures 3D and 3E, ME-200MA0B represents rhCBSΔC enzyme PEGylated with ME-200MA0B, ME-400MA represents rhCBSΔC enzyme PEGylated with ME-400MA, and GL4-400MA represents rhCBSΔC enzyme PEGylated with GL4-400MA.

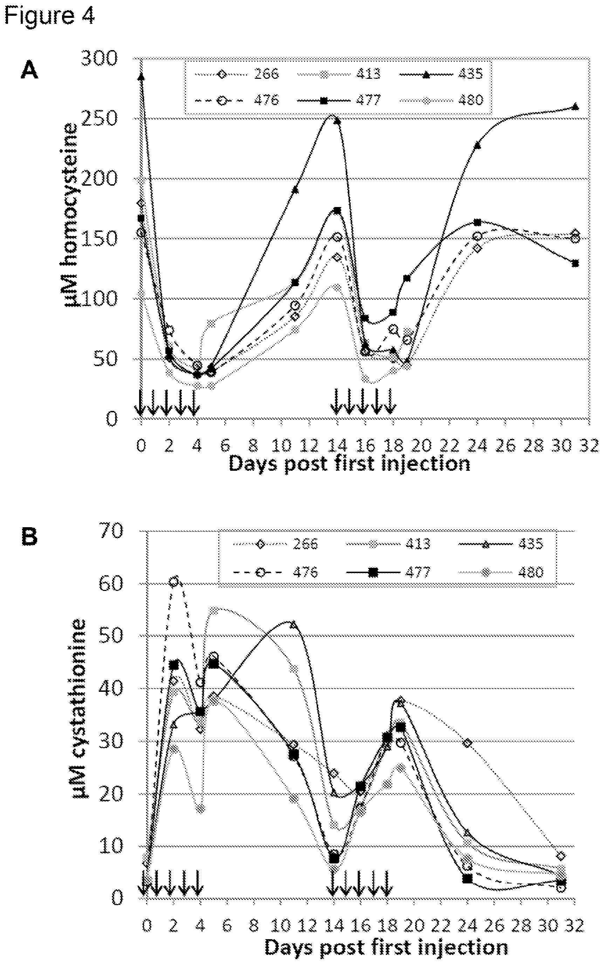

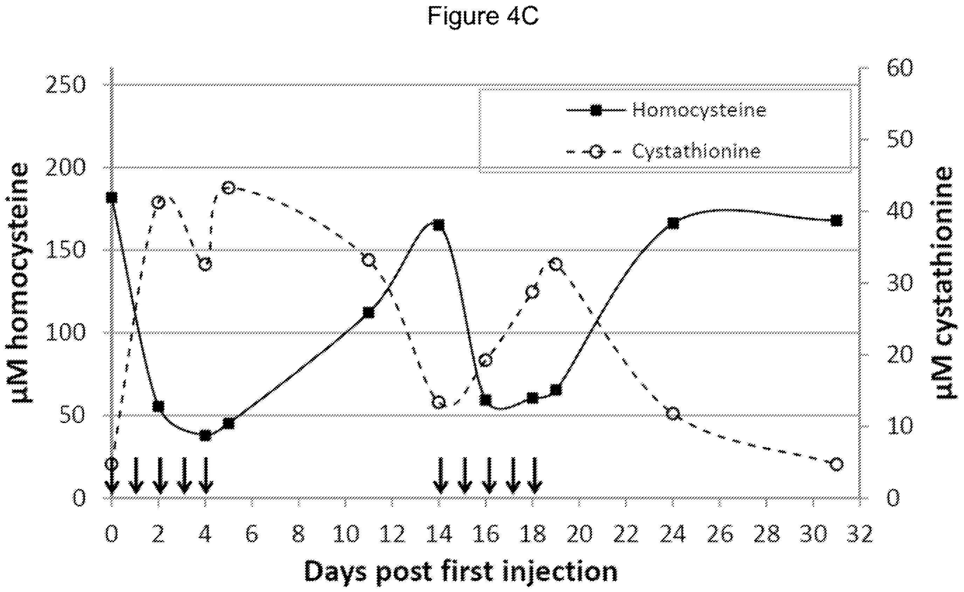

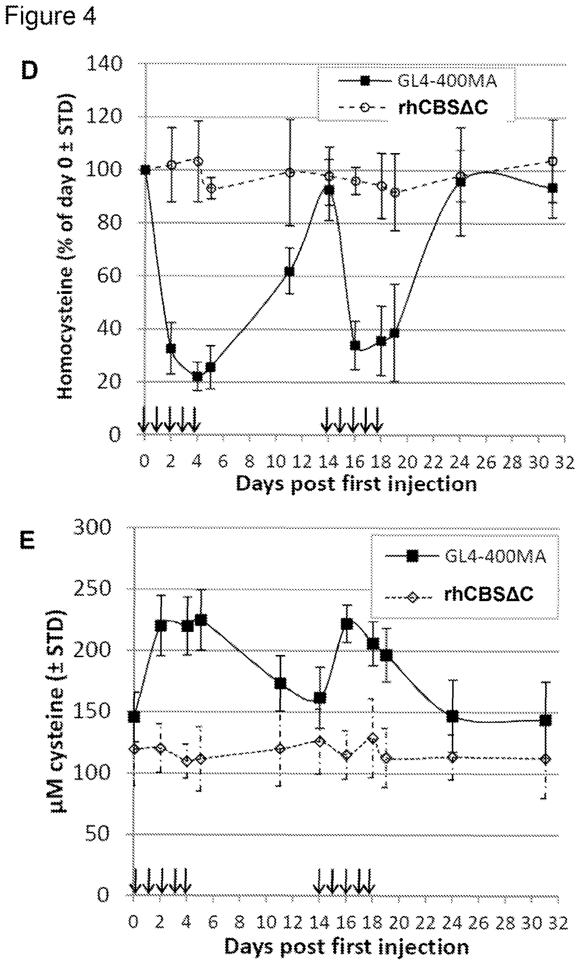

**Figure 4** illustrates experimental evidence that_discontinuing CBS treatment allows amino acid levels to return to pretreatment levels and resuming repeated injection of the PEGylated rhCBSAC significantly impacts homocysteine and cystathionine plasma levels, and restores normal cysteine levels. Six HO mice were injected with rhCBSΔC PEGylated with GL4-400MA PEG, on days 0, 1, 2, 3 and 4, followed by a washout period of 10 days and then again injected on days 14, 15, 16, 17 and 18 (indicated by the arrows). Plasma samples were drawn (always prior to injection) on the following days: 0, 2, 4, 5, 11, 14, 16, 18, 19, 24 and 31. For comparison, the same injection regimen was carried out in 5 HO mice injected with the non-PEGylated enzyme. Plasma metabolite levels were determined as described in Example 1e. **Figure 4a** is a graph showing results for homocysteine, and **Figure 4b** is a graph showing results for cystathionine plasma concentrations for each individual HO mouse. **Figure 4c** is a graph showing average concentrations of homocysteine and cystathionine in plasma from the treated HO mice throughout this study. **Figure 4d** is a graph showing the effect of the PEGylated rhCBSΔC on plasma homocysteine levels as compared to non-PEGylated rhCBSΔC (presented as percentage of time 0) throughout this study. GL4-400MA represents rhCBSΔC enzyme PEGylated with GL4-400MA, and rhCBSAC represents unmodified rhCBSΔC enzyme. **Figure 4e** is a graph showing the effect of PEGylated rhCBSΔC on plasma cysteine levels as compared to non-PEGylated rhCBSΔC throughout this study. GL4-400MA represents rhCBSAC enzyme PEGylated with GL4-400MA, and rhCBSΔC represents unmodified rhCBSΔC enzyme.

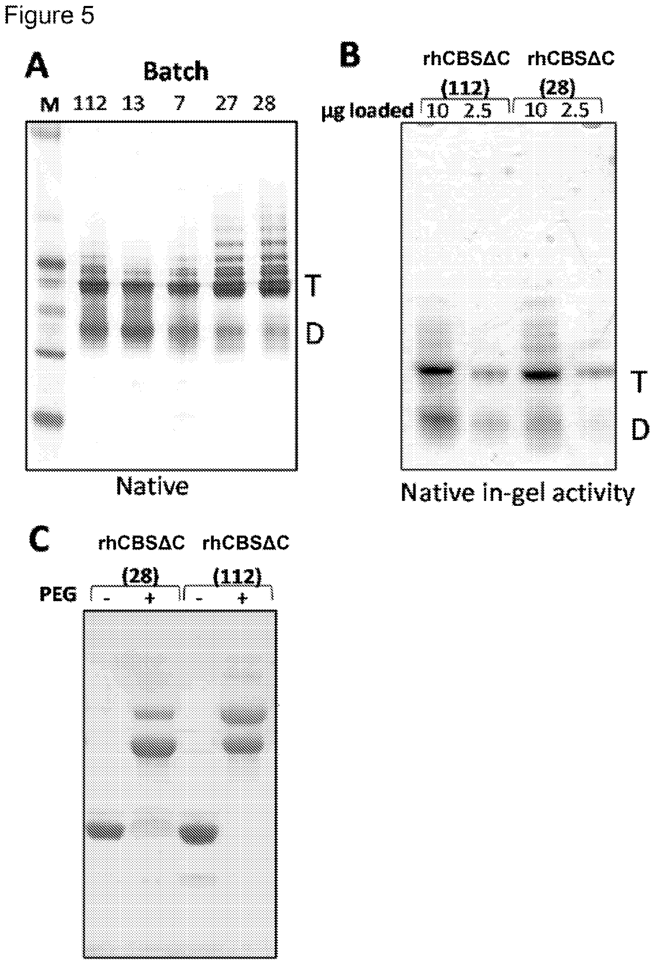

**Figure 5** shows the variability in the degree of aggregation of human truncated CBS preparations (rhCBSΔC). **Figure 5A** is a Coomassie blue-stained 4-15% native gel with different batches (112, 13, 7, 27 and 28) of rhCBSΔC. Note the different ratio between tetrameric form of rhCBSΔC (T) and dimeric form of rhCBSΔC (D), and higher forms of aggregated CBS as well. M, indicates molecular weight markers. **Figure 5B** is a native gel in-gel CBS activity assay showing that the bands that appear on for rhCBSΔC batches 112 and 28 in Fig5A, are indeed CBS related. Again, note the different ratio between tetrameric form of rhCBSΔC (T) and dimeric form of rhCBSΔC (D), and higher forms of aggregated CBS. **Figure 5C** is a SDS PAGE gel (12%) showing PEGylation with ME-200MA0B (+) of two different rhCBSΔC batches showing different ratios between the two PEGylated species (in PEGylated (+) lane) that are formed following PEGylation, while only the unmodified rhCBSAC subunit is present when the PEG reagent was not added (in non-PEGylated (-) lane).

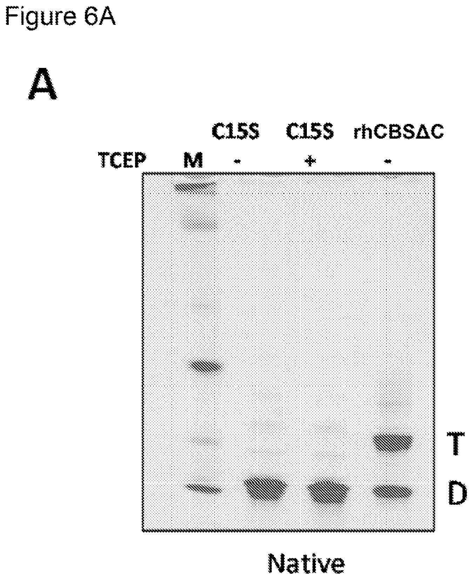

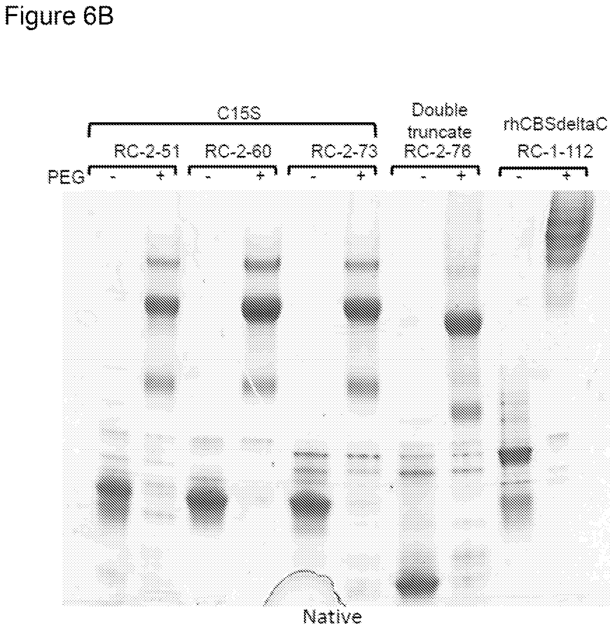

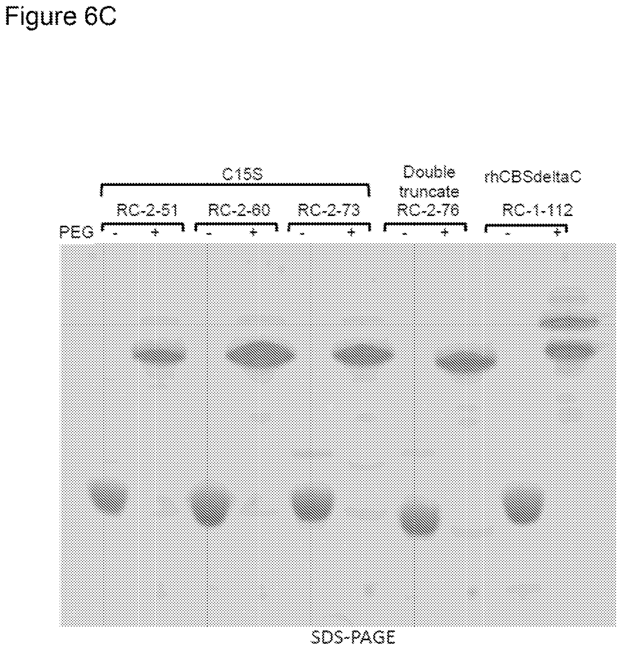

**Figure 6** shows that the human C15S mutant CBS only forms dimers. **Figure 6A** is a native gel that demonstrates that C15S mutant CBS forms only dimers, as opposed to the recombinant human truncated CBS (rhCBSΔC) that forms dimers (D) and tetramers (T) and higher oligomeric forms. TCEP, a reducing agent, does not affect the oligomeric status of C15S mutant. M, indicates molecular weight marker. **Figure 6B** is a native gel that shows different batches (51, 60 and 73) of the C15S mutant CBS demonstrating that human C15S mutant CBS forms reproducibly only dimers as opposed to rtiCBSΔC and recombinant human double truncate (batch RC-2-76; a construct with an additional deletion of residues 2-39 of rhCBSAC) without PEG (-)and following PEGylation with ME-200MA0B (+). PEGylation of either C15S or the double truncated construct gives rise to uniform and reproducible bands, with similar ratios between the PEGylated bands in contrast to rhCBSΔC. **Figure 6C** is the same as Fig 6B, only using a denaturing SDS PAGE. C15S produces a reproducible PEGylation pattern which is different from the recombinant human truncated CBS (rhCBSΔC). This is also comparable to Fig 5C, showing non-reproducible PEGylation patterns with rhCBSΔC.

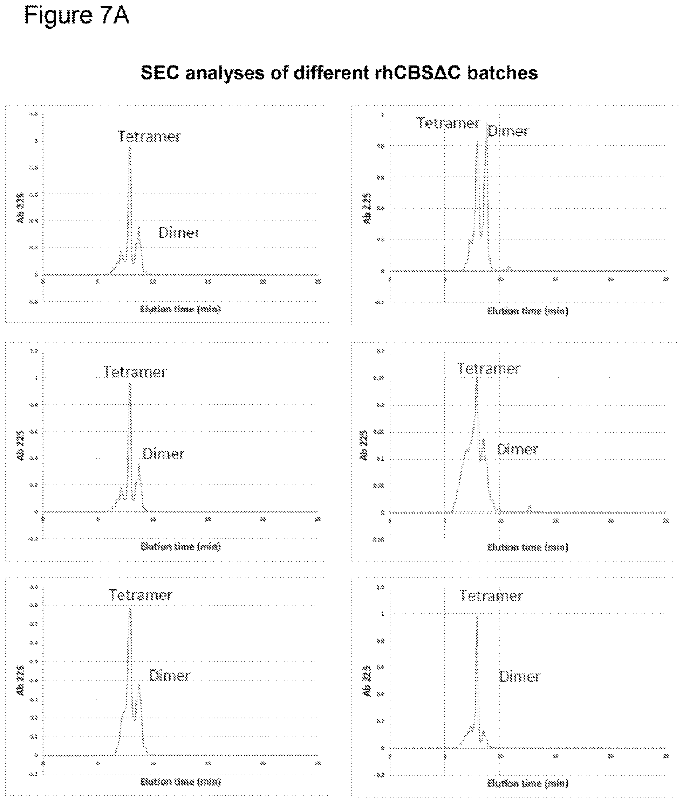

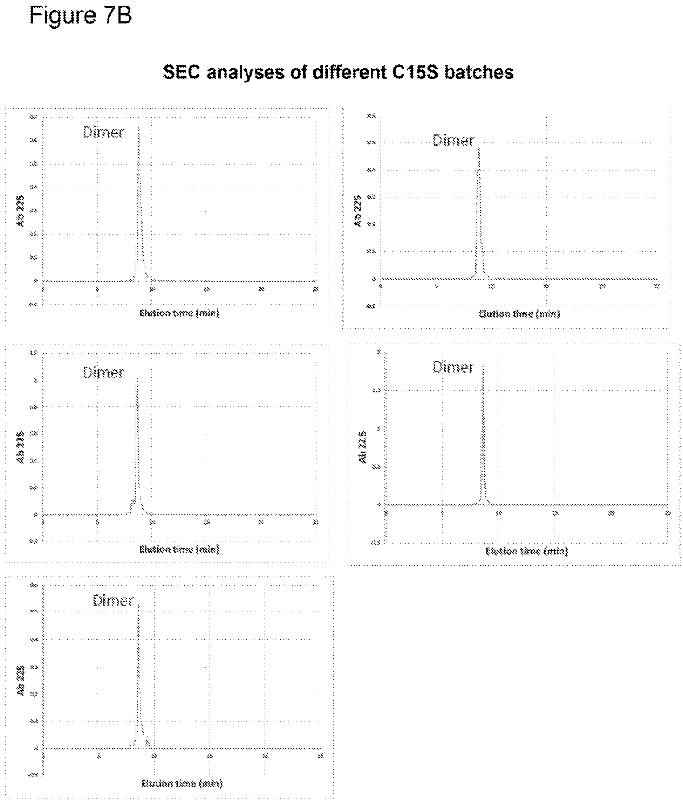

**Figure 7** shows size exclusion chromatography (SEC) analyses of rhCBSAC (Figure 7A) and of the C15S mutant (Figure 7B). Figure 7 provides additional indication that C15S mutant CBS exists solely as a dimer, as it gives a reproducible single peak from five different purification batches (Fig7B). Note the six different patterns of rhCBSΔC in Fig7A and the single peaks of the C15S CBS in Fig7B.

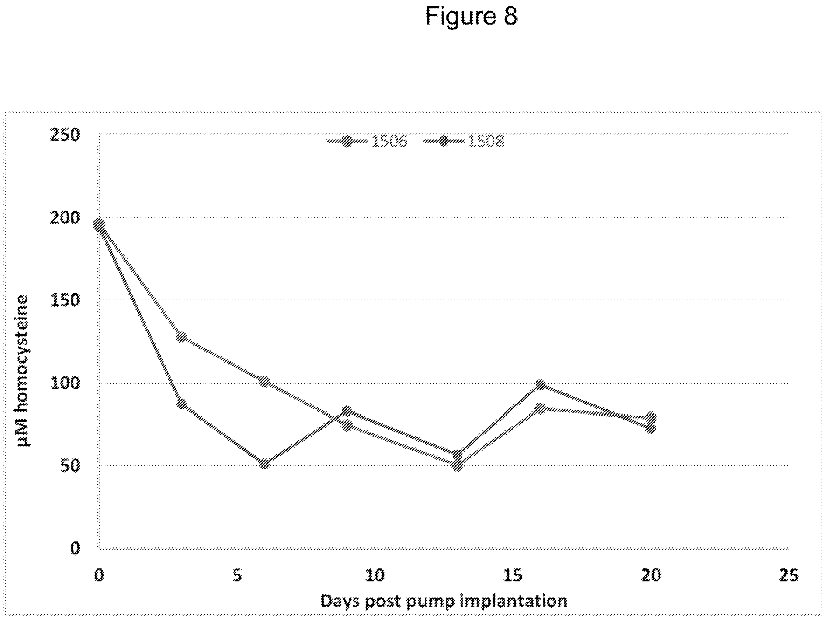

**Figure 8** shows that continuous administration of C15S mutant over 20 days in two HO mice results in a significant and sustained decrease in plasma homocysteine. The C15S mutant enzyme was administrated using the ALZET^{®} pumps to continuously deliver the enzyme to mice instead of repeated subcutaneous injections. ALZET^{®} pump, Model 1002, mean pumping rate 0.21 microliters per hour (of a stock concentration of 26.2mg/mL), mean fill volume 106.6 ul. The mice were bled at the indicated time points, and following isolation of plasma. Hcy was measured by mass spectroscopy.

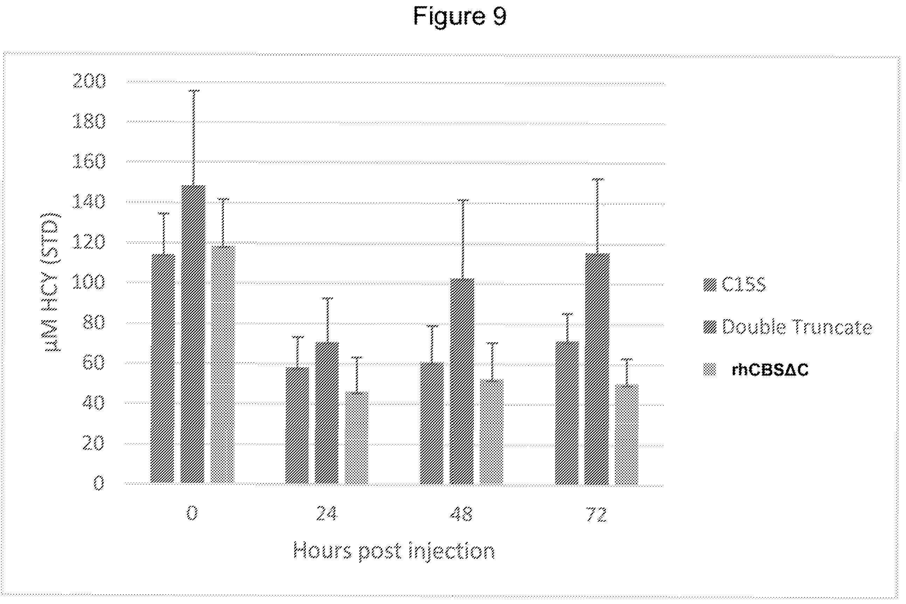

**Figure 9** is a bar graph showing the results of experiments wherein HO mice were injected with a single dose (7.5 mg/kg) at time 0 with rhCBSΔC , C15S mutant (C15S) and the double truncated construct (double truncate) that had been PEGylated with ME-200MA0B. The mice were bled at times 0 (prior to injection), 24, 48 and 72 hours post injection. Levels of plasma homocysteine for each of the groups (n=5-6) are indicated.

### DETAILED DESCRIPTION

Provided herein are forms of human Cystathionine Beta-Synthase (CBS), specifically suitable for pharmaceutical compositions, and methods for treating individuals with homocystinuria, for example, by enzyme replacement therapy (ERT).

The nucleic acid sequence encoding human CBS and the amino acid sequence encoded thereby are available through GenBank Accession No. L19501, and these sequences are also disclosed in U.S. Patent No. 5,523,225, which is incorporated herein by reference in its entirety. The coding sequence for CBS is represented herein as SEQ ID NO: 1, and is a nucleic acid sequence encoding SEQ ID NO: 2, which is the amino acid sequence for full-length human CBS, having 551 amino acid residues. The nucleic acid sequence of the genomic DNA encoding CBS is also publicly available through sequence databases such as GenBank and at University of Colorado-Denver webpage under Kraus Lab.

As used herein, an isolated truncated version of cystathionine β-synthase protein (CBS protein), particularly human CBS protein can include, but is not limited to, purified truncated CBS protein, chemically cleaved and recombinantly produced truncated CBS protein, and isolated CBS protein associated with other proteins. More specifically, an isolated protein, according to the present invention, is a protein (including a polypeptide or peptide) that has been removed from its natural milieu (*i.e*., that has been subject to human manipulation) and can include purified proteins, partially purified proteins, recombinantly produced proteins, and synthetically produced proteins, for example. As such, "isolated" does not reflect the extent to which the protein has been purified. An isolated truncated CBS protein of the present invention can be produced recombinantly in cells, for example bacterial cells. In addition, and by way of example, a "human truncated CBS protein" refers to a truncated CBS protein (as set forth herein) from a human *(Homo sapiens)* or to a CBS protein that has been otherwise produced from the knowledge of the structure (*e*.*g*., sequence) and perhaps the function of a naturally occurring CBS protein from *Homo sapiens*. In other words, a human truncated CBS protein includes biologically active, truncated human CBS protein as described in detail herein.

As used herein, the term "variant" or "mutant" is used to refer to a protein or peptide which differs from a naturally occurring protein or peptide (*i*.*e*., the "prototype" or "wild-type" protein) by changes to the naturally occurring protein or peptide, but which maintains the basic protein and side chain structure of the naturally occurring form. Such changes include, but are not limited to: changes in one, few, or even several amino acid side chains: changes in one, few or several amino acids (*e*.*g*., cysteine at position 15 to serine; C15S); changes in stereochemistry of one or a few atoms, and/or minor derivatizations, including, but not limited to: methylation, glycosylation, phosphorylation, acetylation, myristoylation, prenylation, palmitation, amidation and/or addition of glycosylphosphatidyl inositol. A "variant" or "mutant" can have enhanced, decreased, changed, or essentially similar properties as compared to the naturally occurring protein or peptide. In particular embodiments, the invention provides truncated CBS proteins having C-terminal deletions of the naturally occurring CBS protein.

As used herein, the term "homologue" or "orthologue" refers to protein or DNA sequences with shared ancestry, and can be essentially the same protein or DNA sequence in a different species. Homologous and orthologous proteins and genes often have similar amino acid and nucleotide sequences, respectively, or high sequence similarity (for example, homologous proteins may have 50%, 60%, 70%, 80%, 90%, 95% or greater amino acid sequence similarity).

Methods to measure protein expression levels of CBS according to the invention include, but are not limited to Coomasie blue or silver staining of protein in a separation media, such as gel electrophoresis. Western blotting, immunocytochemistry, other immunologic-based assays; assays based on a property of the protein including but not limited to, enzyme assays, ligand binding or interaction with other protein partners. Binding assays are also well known in the art. For example, a BIAcore instrument can be used to determine the binding constant of a complex between two proteins. The dissociation constant for the complex can be determined by monitoring changes in the refractive index with respect to time as buffer is passed over the chip. Other suitable assays for measuring the binding of one protein to another include, for example, immunoassays such as enzyme linked immunoabsorbent assays (ELISA) and radioimmunoassays (RIA), or determination of binding by monitoring the change in the spectroscopic or optical properties of the proteins through fluorescence, UV absorption, circular dichrosim, or nuclear magnetic resonance (NMR).

In certain aspects, a CBS variant can include any combination of the N-terminal deletions or modifications (*e.g*., deletion of N-terminal residues 2-39 or 1-70) and the C-terminal deletions or modifications (*e.g*., deletion of C-terminal residues 383-551, 397-551, 414-551, 442-551, 489-551, 497-551, 524-551, 534-551 or 544-551) described herein or in U.S. Patent No's. 7,485,307 and 8,007,787, both incorporated by reference herein. In another embodiment additional modifications can be achieved by modification of other amino acid residues to provide a given percent identity to the wild-type CBS sequence, In particular embodiments, the human CBS variant of the invention is a truncated recombinant human CBS (rhCBSΔC) homodimeric enzyme wherein the C-terminal regulatory region has been removed (SEQ ID NO: 3). In other embodiments, the human CBS variant of the invention is a rhCBSΔC wherein the cysteine at amino acid position 15 has been mutated to serine (C15S) (SEQ ID NO: 13).

In other embodiments of the invention, any of the CBS variants described herein has no more than one or two non-CBS amino acid residues at the N-terminus (*i*.*e*., the variant comprises no more than one or two amino acid residues at the N-terminus that is/are not a residue of the naturally occurring human cystathionine β-synthase amino acid sequence at that position). Such a variant can be produced, for example, using the novel method of recombinant CBS production described below.

In further embodiments any of the above-described CBS variants of the present invention, including any truncated CBS protein, comprises an amino acid sequence that is at least about 50% identical, or at least about 55% identical, or at least about 60% identical, or at least about 65% identical, or at least about 70% identical, or at least about 75% identical, or at least about 80% identical, or at least about 85% identical, or at least about 90% identical, or at least about 95% identical, or at least about 96% identical, or at least about 97% identical, or at least about 98% identical, or at least about 99% identical, to the wild-type amino acid sequence represented by SEQ ID NO:2, or to a biologically active truncation thereof, including *inter alia* non-heme-binding or non-S-adenosylmethionine (AdoMet) binding, wherein said variants remain catalytically active (for example, wherein certain truncated forms of CBS can be even more active than the full length CBS in the presence of AdoMet). In particular embodiments, the human CBS variant of the invention is a truncated recombinant human CBS (rhCBSΔC) homodimeric enzyme wherein the C-terminal regulatory region has been removed (SEQ ID NO: 3). In other embodiments, the human CBS variant of the invention is a truncated recombinant human CBS enzyme wherein the cysteine at amino acid position 15 has been mutated to serine (SEQ ID NO: 13).

In certain embodiments, a CBS protein of the present invention comprises an amino acid sequence that is less than 100% identical to SEQ ID NO: 2, particularly truncated embodiments thereof having an amino acid sequence of the truncation variants as set forth in U.S. Patent No. 8,007,787 and particularly SEQ ID NO: 3 and SEQ ID NO: 13 set forth herein, and in specific embodiments having less than 99% sequence identity, less than 98% sequence identity, less than 97% sequence identity, less than 96% sequence identity, less than 95% sequence identity, less than 94% sequence identity, less than 93% sequence identity, less than 92% sequence identity, less than 91% sequence identity, less than 90% sequence identity, and so on, in increments of the truncation variants as set forth in U.S. Patent No. 8,007,787 and particularly SEQ ID NO: 3 and SEQ ID NO: 13 set forth herein.

As used herein, unless otherwise specified, reference to a percent (%) identity refers to an evaluation of homology which is performed using a sequence alignment tool or program, including but not limited to (1) a BLAST 2.0 Basic BLAST homology search using blastp for amino acid searches and blastn for nucleic acid searches with standard default parameters, wherein the query sequence is filtered for low complexity regions by default; (2) a BLAST 2 alignment (using the parameters described below); (3) and/or PSI-BLAST with the standard default parameters (Position-Specific Iterated BLAST. It is noted that due to some differences in the standard parameters between BLAST 2.0 Basic BLAST and BLAST 2, two specific sequences might be recognized as having significant homology using the BLAST 2 program, whereas a search performed in BLAST 2.0 Basic BLAST using one of the sequences as the query sequence may not identity the second sequence in the top matches. In addition, PSI-BLAST provides an automated, easy-to-use version of a "profile" search, which is a sensitive way to look for sequence homologues. The program first performs a gapped BLAST database search. The PSI-BLAST program uses the information from any significant alignments returned to construct a position-specific score matrix, which replaces the query sequence for the next round of database searching. Therefore, it is to be understood that percent identity can be determined by using anyone of these programs,

CBS derivatives are included in the scope of the present invention. Such derivatives are chemically modified CBS polypeptide compositions in which CBS polypeptide is linked to a polymer. The polymer selected is typically water-soluble so that the protein to which it is attached does not precipitate in an aqueous environment and so that the CBS enzyme is biologically active in biological fluids, such as a physiological environment. The polymer may be of any molecular weight, and may be branched or unbranched. Included within the scope of CBS polypeptide polymers is a mixture of polymers. In specific embodiments, for therapeutic use of the end-product preparation, the polymer will be pharmaceutically acceptable.

The water soluble polymer or mixture thereof may be selected from the group consisting of, for example, polyethylene glycol (PEG), monomethoxy-polyethylene glycol, dextran (such as low molecular weight dextran, of, for example about 6 kDa), cellulose, or other carbohydrate based polymers, poly-(N-vinyl pyrrolidone) polyethylene glycol, propylene glycol homopolymers, a polypropylene oxide/ethylene oxide co-polymer, polyoxyethylatedpolyols (*e.g*., glycerol), polysalicylic acid, and polyvinyl alcohol. Also encompassed by the invention are bifunctional PEG cross-linking molecules that may be used to prepare covalently attached CBS polypeptide multimers. In an embodiment the polyethylene glycol molecules are selected from unbranched and branched polyethylene glycol, wherein the polyethylene glycol molecules have a molecular weight that equals or exceeds 2000 Daltons. In another embodiment, the polyethylene glycol molecules have a molecular weight in the range of 2 - 100kD, 5 - 80 kD or 10 - 40 kD. In yet another embodiment, the polyethylene glycol molecule is selected from ME-200MA0B, ME-020MA, ME-400MA, GL2-400MA. GL4-400MA, GL2-800MA, ME-050GS, ME-200GS, ME-200AL or MEPA-20T.

In particular embodiments, the invention provides a truncated recombinant human CBS (rhCBSΔC) homodimeric enzyme wherein the C-terminal regulatory region has been removed (SEQ ID NO: 3) that has been chemically modified by covalent linkage with polyethylene glycol (PEG), comprising the "PEGylated species" set forth herein. In other embodiments of the invention, the human CBS variant of the invention is a truncated recombinant human CBS enzyme wherein the cysteine at amino acid position 15 has been mutated to serine (SEQ ID NO: 13) and that has been chemically modified by covalent linkage with polyethylene glycol (PEG), comprising the "PEGylated species" set forth herein.

Specific embodiments of the various PEG reagents used in the PEGylation reactions to generate the modified PEGylated species are identified in Table 1.

**TABLE 1**

| **PEG Designation** | **Target groups** | **Molecular weight (daltons)** |
|---|---|---|
| ME-200MA0B | -SH | 20,000 |
| ME-020MA | -SH | 2,000 |
| ME-400MA | -SH | 40,000 |
| GL2-400MA | -SH | 40,000 |
| GL4-400MA | -SH | 40,000 |
| GL2-800MA | -SH | 80,000 |
| ME-050GS | -NH₂, -OH, -OH | 5,000 |
| ME-200GS | -NH₂, -OH, -SH | 20,000 |
| ME-200AL | -NH₂ | 20,000 |
| MEPA-20T | -COOH | 20,000 |

PEGylation of CBS polypeptides may be carried out by any of the PEGylation reactions known in the art. PEGylation may be carried out via an acylation reaction or an alkylation reaction with a reactive polyethylene glycol molecule (or an analogous reactive water-soluble polymer) as described below. For the acylation reactions, the polymer(s) selected should have a single reactive ester group. For reductive alkylation, the polymer(s) selected should have a single reactive aldehyde group. A reactive aldehyde is, for example, polyethylene glycol propionaldehyde, which is water stable, or mono C₁-C₁₀ alkoxy or aryloxy derivatives thereof. In an embodiment, the polyethylene glycol molecules are attached to CBS via an attachment group selected from N-Hydroxysuccinimide (NHS), amine, and aldehyde consisting of a monoaldehyde, a monoester of a monoacid, a monoamine, a monothiol, a monodisulfide, a monobromophenyl carbonate, a monochlorophenyl carbonate, a monofluorophenyl carbonate, a mononitrophenyl carbonate, a monocarbonylimidazole, a monohydrazide, a monoiodoacetamide, a monomaleimide, a monoorthopyridyl disulfide, a monooxime, a monophenyl glyoxal, a monothiazolidine-2-thione, a monothioester, a monotriazine and a monovinylsulfone.

One water-soluble polymer for use herein is polyethylene glycol, abbreviated PEG. As used herein, polyethylene glycol is meant to encompass any of the forms of PEG that have been used to derivatize other proteins, such as mono-(C₁-C₁₀) alkoxy- or aryloxy-polyethylene glycol.

In general, chemical derivatization may be performed under any suitable conditions used to react a biologically active substance with an activated polymer molecule. Methods for preparing PEGylated CBS polypeptides will generally comprise the steps of (a) reacting the polypeptide with polyethylene glycol (such as a reactive ester, amine, aldehyde or maleimide derivative of PEG) under conditions whereby CBS polypeptide becomes attached to one or more PEG groups, and (b) obtaining the reaction product(s). In general, the optimal reaction conditions for the acylation reactions will be determined based on known parameters and the desired result. For example, the larger the ratio of PEG: protein, the greater the percentage of poly-PEGylated product. In one particular aspect, the CBS polypeptide derivative will have a single PEG moiety at the amino terminus. In particular embodiments, the PEGylated CBS enzyme provided by the invention has an average of about 1 to about 10, more particularly 2 to about 5 and more particularly 3 to 5 PEG molecules covalently attached to each enzyme subunit in the composition.

Proteins of the present invention are preferably retrieved, obtained, and/or used in "substantially pure" form. As used herein, "substantially pure" refers to a purity that allows for the effective use of the protein *in vitro*, *ex vivo* or *in vivo* according to the present invention. For a protein to be useful in an *in vitro*, *ex vivo* or *in vivo* method according to the present invention, it is substantially free of contaminants, other proteins and/or chemicals that might interfere of that would interfere with its use in a method disclosed by the present invention, or that at least would be undesirable for inclusion with an CBS protein (including homologues) when it is used in a method disclosed by the present invention. Such methods include enzymatic reactions (*e.g*., production of cystathionine), preparation of therapeutic compositions, administration in a therapeutic composition, and all other methods disclosed herein. A "substantially pure" protein, as referenced herein, is a protein that can be produced by any method (*i*.e., by direct purification from a natural source, recombmantly, or synthetically), and that has been purified from other protein components such that the protein comprises at least about 80% weight/weight of the total protein in a given composition (*e.g*., the CBS protein is about 80% of the protein in a solution/composition/buffer), and more preferably, at least about 85%, and more preferably at least about 90%, and more preferably at least about 91 %, and more preferably at least about 92%, and more preferably at least about 93%, and more preferably at least about 94%, and more preferably at least about 95%, and more preferably at least about 96%, and more preferably at least about 97%, and more preferably at least about 98%, and more preferably at least about 99%, weight/weight of the total protein in a given composition. In embodiments of the CBS proteins or truncated variants thereof produced in recombinant bacteria, the terms "purified" or "substantially pure" will be understood to encompass purification from lipopolysaccharides and other pyrogenic compounds.

It will be appreciated by one skilled in the art that use of recombinant DNA technologies can improve control of expression of transfected nucleic acid molecules by manipulating, for example, the number of copies of the nucleic acid molecules within the host cell, the efficiency with which those nucleic acid molecules are transcribed, the efficiency with which the resultant transcripts are translated, and the efficiency of posttranslational modifications. Additionally, the promoter sequence might be genetically engineered to improve the level of expression as compared to the native promoter.

Recombinant techniques useful for controlling the expression of nucleic acid molecules include, but are not limited to, integration of the nucleic acid molecules into one or more host cell chromosomes, addition of vector stability sequences to plasmids, substitutions or modifications of transcription control signals (*e.g*., promoters, operators, enhancers), substitutions or modifications of translational control signals (*e.g*., ribosome binding sites, Shine-Dalgarno sequences), modification of nucleic acid molecules to correspond to the codon usage of the host cell, and deletion of sequences that destabilize transcripts.

In a further aspect, the present invention relates to a method to recombinantly produce and purify a human cystathionine β-synthase. The method includes the step of cloning a nucleic acid sequence encoding a human CBS enzyme or a truncated or mutated variant thereof as set forth in U.S. Patent No. 8,007,787 and specifically in SEQ ID NO: 4 herein into an expression vector. In certain embodiments the human CBS-encoding sequence has been modified to utilize codons optimized for expression in a microorganism such as *E. coli.* In other embodiments the vector includes: (a) a cloning site that will link a fusion partner (*e.g.,* glutathione S-transferase, or GST) to the nucleic acid sequence to be expressed, and (b) a protease cleavage recognition site for the human rhinovirus 3C protease (*e.g.,* available from GE Healthcare as a fusion protein called PreScission protease) or for a protease using a similar cleavage site, for cleaving the fusion partner from the CBS protein after expression of the recombinant fusion protein. As part of the invention, the expression vector is first genetically modified for the specific introduction of a CBS-encoding nucleic acid sequence which will result in expression of a CBS-fusion protein that can be cleaved by the human rhinovirus 3C protease such that a CBS protein having only one additional non-CBS, N-terminal amino acid residue is produced. This result is not possible using the unmodified multiple cloning site in the commercially available vector. The CBS-encoding nucleic acid sequence is introduced into the genetically modified vector, the recombinant fusion protein is expressed and purified using conventional methods or those suitable for CBS production (*see e.g.* U.S. Patent No. 5,635,375, s*upra)*, and finally, the fusion partner and all but one of the non-CBS amino acid residues is cleaved from the CBS protein, leaving a highly purified, nearly completely human recombinant CBS protein which is ideal for use in human therapeutic applications. In particularly advantageous embodiments, the nucleotide encoding human CBS protein is engineered to the codon usage frequency of the recombinant cell in which it is recombinantly made. A non-limiting example of such an embodiment is set forth herein as SEQ ID NO: 4, wherein the CBS-encoding nucleic acid has been adapted for optimum recombinant expression in *E. coli.*

Some aspects of the present invention include a composition comprising any of the CBS variants described herein for *in vitro* cystathionine or cysteine production, to remove or produce hydrogen sulfide *in vitro*, or for therapeutic uses *in vivo* (*e.g.,* to treat or prevent homocystinuria and conditions related thereto). Therefore, another embodiment of the invention relates to a composition comprising an isolated CBS protein and in particular a truncated variant thereof as set forth in U.S. Patent No. 8,007,787 and specifically in SEQ ID NO: 3 or SEQ ID NO: 13 as described herein. The composition typically also includes a pharmaceutically acceptable carrier. The compositions and their components can be used in any of the *in vitro* or therapeutic embodiments of the invention described herein.

As used herein, "HO" mice are a new mouse model of classical homocystinuria in which the mouse cbs gene is inactivated and that exhibits low-level expression of the human CBS transgene under the control of the human CBS promoter. This mouse model exhibits severe elevations in both plasma and tissue levels of Hey, methionine, S-adenosylmethionine, and S-adenosylhomocysteine and a concomitant decrease in plasma and hepatic levels of cysteine. See Maclean et al., 2010 Mol. Genet. Metab. 101:153-62).

Compositions of the present invention are useful for producing cystathionine and cysteine *in vitro* or for treating an individual that will benefit from increased CBS activity (*e.g.,* an individual with homocystinuria).

According to the present invention, a "pharmaceutically acceptable carrier" includes pharmaceutically acceptable excipients and/or pharmaceutically acceptable delivery vehicles, which are suitable for use in administration of the composition to a suitable *in vitro, ex vivo* or *in vivo* site. A suitable *in* vitro, *in vivo* or *ex vivo* site is preferably any site where it is desirable to regulate CBS activity. Pharmaceutically acceptable carriers are capable of maintaining a protein or recombinant nucleic acid molecule of the present invention in a form that, upon arrival of the protein or recombinant nucleic acid molecule at the target cell or tissue in a culture or in an individual, the protein or recombinant nucleic acid molecule is capable of interacting with its target (*e.g.,* a substrate for CBS).

Suitable excipients of the present invention include excipients or formularies that transport or help transport, but do not specifically target a composition to a cell (also referred to herein as non-targeting carriers). Examples of pharmaceutically acceptable excipients include, but are not limited to water, phosphate buffered saline. Ringer's solution, dextrose solution, serum-containing solutions, Hank's solution, other aqueous physiologically balanced solutions, oils, esters and glycols. Aqueous earners can contain suitable auxiliary substances required to approximate the physiological conditions of the recipient, for example, by enhancing chemical stability and isotonicity. Compositions of the present invention can be sterilized by conventional methods and/or lyophilized.

One type of pharmaceutically acceptable carrier includes a controlled release formulation that is capable of slowly releasing a composition of the present invention into an individual or culture. As used herein, a controlled release formulation comprises a compound of the present invention (*e.g.*, a protein (including homologues), an antibody, a nucleic acid molecule, or a mimetic) in a controlled release vehicle. Suitable controlled release vehicles include, but are not limited to, biocompatible polymers, other polymeric matrices, capsules, microcapsules, microparticles, bolus preparations, osmotic pumps (e.g, ALZET^{®} osmotic pump), diffusion devices, liposomes, lipospheres, and transdermal delivery systems. Other carriers of the present invention include liquids that, upon administration to an individual, form a solid or a gel *in situ.* In specific embodiments, carriers are also biodegradable (*i.e.*, bioerodible). When the compound is a recombinant nucleic acid molecule, suitable carriers include, but are not limited to liposomes, viral vectors or other carriers, including ribozymes, gold particles, poly-L-lysine/DNA-molecular conjugates, and artificial chromosomes. Natural lipid-containing carriers include cells and cellular membranes. Artificial lipid-containing carriers include liposomes and micelles.

A carrier of the present invention can be modified to target to a particular site in an individual, thereby targeting and making use of a protein of the present invention at that site. A pharmaceutically acceptable carrier that is capable of targeting can also be referred to herein as a "delivery vehicle" or "targeting carrier". Suitable modifications include manipulating the chemical formula of the lipid portion of the delivery vehicle and/or introducing into the vehicle a targeting agent capable of specifically targeting a delivery vehicle to a preferred site or target site, for example, a preferred cell type. A "target site" refers to a site in an individual to which one desires to deliver a composition. Alternatively, said pharmaceutically acceptable carriers can comprise agents suitable for delivering said CBS proteins to an animal, preferably a human, in blood plasma or serum. Suitable targeting compounds include ligands capable of selectively (*i.e.*, specifically) binding another molecule at a particular site. Examples of such ligands include antibodies, antigens, receptors and receptor ligands. Manipulating the chemical formula of the lipid portion of the delivery vehicle can modulate the extracellular or intracellular targeting of the delivery vehicle. For example, a chemical can be added to the lipid formula of a liposome that alters the charge of the lipid bilayer of the liposome so that the liposome fuses with particular cells having particular charge characteristics. In specific embodiments, liposomes of the present invention include those liposomes commonly used in, for example, protein delivery methods known to those of skill in the art. Complexing a liposome with a protein of the present invention can be achieved using methods standard in the art.

In yet another aspect, the present invention relates to a method to regulate biological processes, including cystathionine production, by regulating the expression and/or activity of CBS. This embodiment can generally include the use (e.g., administration) of therapeutic compositions comprising one or more of the CBS variants, particularly truncated CBS variants thereof as set forth in U.S. Patent No. 8,007,787 and specifically in SEQ ID NO: 3 or SEQ ID NO: 13 as described herein that are useful in a method of regulating the production of cystathionine that are mediated by or associated with the expression and biological activity of CBS.

Accordingly, in one embodiment, the method of the present invention regulates cystathionine production in an animal or human individual, wherein the individual is protected from or treated for a disease that is amenable to regulation of cystathionine production, such as homocystinuria and conditions/symptoms related thereto (*e.g.*, dislocated optic lenses, skeletal disorders, mental retardation and premature arteriosclerosis and thrombosis). As used herein, the phrase "protected from a disease" refers to reducing the symptoms of the disease; reducing the occurrence of the disease, and/or reducing the severity of the disease. Protecting an individual can refer to the ability of a therapeutic composition of the present invention, when administered to an individual, to prevent a disease from occurring and/or to cure or to treat the disease by alleviating disease symptoms, signs or causes. As such, to protect an individual from a disease includes both preventing disease occurrence (prophylactic treatment) and treating an individual that has a disease or that is experiencing initial symptoms or later stage symptoms of a disease (therapeutic treatment). The term, "disease" refers to any deviation from the normal health of an individual and includes a state when disease symptoms are present, as well as conditions in which a deviation (*e.g*., in non-limiting examples including infection, gene mutation, and genetic defect, *etc.*) has occurred, but symptoms are not yet manifested (e.g., a predisease condition).

More specifically, a therapeutic composition as described herein, when administered to an individual by the method of the present invention, preferably produces a result which can include alleviation of the disease (e.g., reduction of at least one symptom or clinical manifestation of the disease), elimination of the disease, alleviation of a secondary disease resulting from the occurrence of a primary disease, or prevention of the disease. In an additional aspect, administration of the therapeutic composition can produce a result that can include increased accumulation of downstream metabolites of transsulfuration in a mammal.

According to the present invention, an effective administration protocol (*i.e*., administering a therapeutic composition in an effective manner) comprises suitable dose parameters and modes of administration that result in the desired effect in the individual (e.g., an increase in the activity of cystathionine β-synthase in the individual or an increase in the condensation of serine and homocysteine to form cystathionine, preferably so that the individual is protected from the disease (*e.g*., by disease prevention or by alleviating one or more symptoms of ongoing disease). Effective dose parameters can be determined using methods standard in the art for a particular disease. Such methods include, for example, determination of survival rates, side effects (*i.e.*, toxicity) and progression or regression of disease.

In accordance with the present invention, a suitable single dose size is a dose that results in an increase of CBS activity or control of Hey levels for an individual with normal protein intake or formation of cystathionine or cysteine in an individual, or in the amelioration of at least one symptom of a condition in the individual, when administered one or more times over a suitable time period. Doses can vary depending upon the disease being treated. One of skill in the art can readily determine appropriate single dose sizes for a given individual based on the size of an individual and the route of administration.

In certain embodiments of the invention, a suitable single dose of a therapeutic composition of the present invention is an amount that, when administered by any route of administration, increases CBS activity as described above, as compared to an individual which has not been administered with the therapeutic composition of the present invention (*i.e*., a pre-determine control individual or measurement), as compared to the individual prior to administration of the composition, or as compared to a standard established for the particular disease, individual type and composition. The administered therapeutic composition is enzymatically active for an extended period of time. This period of time preferably exceeds 24 hours. The chemically modified therapeutic composition retains 70% or more of the initial activity of the administered composition after 24 hours. More preferably the period of enzymatic effective activity of the administered therapeutic composition exceeds 48 hours. Even more preferably the period of effective enzymatic activity of the administered therapeutic composition exceeds 72 hours. In certain embodiments, the therapeutic composition may be administered several (*e.g*., twice or three or more) times per day or less frequently, including but not limited to weekly, biweekly, monthly, bimonthly or less frequently. In other embodiments, the therapeutic composition may be administered continuously over the course of days, weeks or months. In another embodiment of the invention, a suitable dose of the therapeutic composition of the invention can administered in conjunction with betaine (such as CYSTADANE^{®}), a much relaxed protein restricted diet, an anti-coagulant, or a statin.

As discussed above, a therapeutic composition of the present invention is administered to an individual in a manner effective to deliver the composition to a cell, a tissue, and/or systemically to the individual, whereby the desired result is achieved as a result of the administration of the composition. Suitable administration protocols include any *in vivo* or *ex vivo* administration protocol. The preferred routes of administration will be apparent to those of skill in the art, depending on the type of condition to be prevented or treated; whether the composition is nucleic acid based, protein based, of cell based; and/or the target cell/tissue. For proteins, methods of *in vivo* administration include parenteral administration, specifically including but not limited to, osmotic pump administration, intravenous administration, intraperitoneal administration, intramuscular administration, intranodal administration, intracoronary administration, intraarterial administration (*e.g.*, into a carotid artery), subcutaneous administration, intraarticular administration, intraventricular administration, and direct injection into a tissue. Combinations of routes of delivery can be used and in some instances, may enhance the therapeutic effects of the composition.

Many of the above-described routes of administration, including intravenous, intraperitoneal, intradermal, intramuscular administrations or through osmotic pumps can be performed using methods standard in the art.

One method of local administration is by direct injection. Direct injection techniques are particularly useful for administering a composition to a cell or tissue that is accessible by surgery, and particularly, on or near the surface of the body. Administration of a composition locally within the area of a target cell refers to injecting the composition centimeters and preferably, millimeters from the target cell or tissue.

Another method of local administration is through the use of one or more osmotic pumps (*e.g.*, ALZET^{®} osmotic pump), which allow for a steady delivery of a drug for a prolonged period of time (*e.g.*, weeks or months). Implanted osmotic pumps are particularly useful for administering a composition to a cell, tissue or subject that is accessible by surgery, and particularly, on or near the surface of the body. Administration of a composition locally within the area of a target cell or tissue of a subject refers to implanting the osmotic pump containing the composition centimeters and preferably, millimeters from the target cell or tissue.

In the method of the present invention, therapeutic compositions can be administered to any member of the Vertebrate class, Mammalia, including, without limitation, primates, rodents, livestock and domestic pets. Livestock include mammals to be consumed or that produce useful products (e.g., sheep for wool production). Preferred individuals to protect include humans.

Some aspects of the present invention include use of the isolated CBS polypeptide or any of the CBS variants described herein for the making non-aggregating CBS derivatives.

Each reference described and/or cited herein is incorporated by reference in its entirety.

The following examples are provided for the purpose of illustration and are not intended to limit the scope of the present invention.

### EXAMPLES

### Example 1: Production of Truncated CBS Protein in Bacteria

### a. Recombinant Expression of Truncated CBS Protein in Bacteria

A truncated human CBS variant lacking specific portions of the non-conserved regions (rhCBSΔC; SEQ ID No: 3) was constructed and over-expressed using the previously described *E. coli* based expression system (Kozich and Kraus, 1992, *supra*). Constructs encoding the truncated human CBS protein variant rhCBSΔC (DNA encoding rhCBSΔC is shown in SEQ ID NO: 4) were generated by a modification of the previously described pHCS3 CBS expression construct (Kozich and Kraus, 1992 Hum. Mutat. 1:113-123) which contains the CBS full-length coding sequence (SEQ ID NO: 1) cloned into pKK388.1. To generate C-terminal deletion constructs, CBS cDNA fragments spanning the desired nucleotide residues were amplified using primers incorporating Sph I and Kpn I sites to the 5' and 3' respective ends of the PCR product. All PCR products were then cut with Sph I and Kpn I and cloned by ligation into the pHCS3 vector digested with Sph I and Kpn I. An Sph I site naturally occurs in the CBS cDNA just upstream of the antisense primer hybridization site (base pair position 1012, according to the CBS cDNA numbering SEQ ID NO: 1). PCR products thus generated were then digested with Nco I and Sph I and ligated into the pHCS3 plasmid cut with the same enzymes.
pKK CBS A414-5 51
Sense: 5'-CGTAGAATTCACCTTTGCCC**GCATGC**TGAT (SEQ ID NO: 51 (*SphI restriction site in bold*)
Antisense: 5'-TACG**GGTACC**TCAACGGAGGTGCCACCACCAGGGC (SEQ ID NO: 6) (*KpnI restriction site in bold*)

Finally, the construct was transformed into *E.coli* BL21 (Stratagene). The authenticity of the construct was verified by DNA sequencing using a Thermo Sequenase Cy5.5 sequencing kit (Amersham Pharmacia Biotech) and the Visible Genetics Long-Read Tower System-V3.1 DNA sequencer according to the manufacturer's instructions.

For bacterial expression analysis of CBS deletion mutants, growth of *E. coli* BL21 cells bearing the CBS truncation mutant construct, induction of expression and the generation of crude cell lysates were performed as described previously (Maclean et al., 2002, Hum. Mutat. 19:641-55).

An alternative method was also employed to make a sequence-optimized, truncated human CBS enzyme (rhCBSΔC; SEQ ID No: 3) in a pET28a (+) vector. The full length (551 aa) human CBS coding sequence was optimized for bacterial expression and cloned into the pUC57 vector following digestion with the *Eco*RV restriction enzyme, by GenScript USA Inc. (NJ, USA). The CBS sequence was then amplified by PCR using primers A1 and A2 to generate a sequence coding for the truncated enzyme (aa 1-413):

### Primers:

**A1**
   *5'agtcgc*CCATG**G**cgtcagaaaccccgcag 3' (SEQ ID NO: 7) *NcoI restriction site in CAPs. Bold G mutated to C (for proline).*
**A2**
   5' *atcgcg*CTCGAGttagcgcaggtgccaccac *3'* (SEQ ID NO: 8) *XhoI restriction site in CAPs, followed by TTA for stop codon*

The PCR product was then digested with the restriction enzymes *Nco*I and *Xho*I, and ligated into the pET-28a(+) vector (commercially available from EMD Millipore, Billerica, MA) that was digested with the same enzymes. Cloning into the optimal *Nco*I site of pET-28a(+) results in a G→C mutation as compared to the CBS wild-type sequence (coding for alanine instead of proline). Accordingly, a Site-Directed Mutagenesis kit (Stratagene, CA, USA) utilizing primers B1 and B2 was used to re-generate the wild type sequence to code for proline:
**B1**
   5' GGAGATATACCATG**C**CGTCAGAAACCCCGC 3' (SEQ ID NO: 9)
**B2**
   5' GCGGGGTTTCIGACG**G**CATGGTATATCTCC 3' (SEQ ID NO: 10)

The same strategy was used to generate the C15S mutant (T→A mutation) by using primers:
**C1** 5'- TGGGTCC'GACCGGT**A**GCCCGCAC -3' (SEQ ID NO: 11) and
**C2** 5' - GTGCGGGC**T**ACCCCTCGGACCCA - 3' (SEQ ID NO: 12).

The full sequences of both the optimized, rhCBSΔC and the C15S mutant polynucleotide constructs, were verified by sequencing.

Expression of the truncated CBS is controlled by the upstream T7 promoter in the pET-28a(+) vector, and requires transformation into DES bacteria and induction by IPTG.

### b. Expression of the sequence-optimized, truncated human CBS enzyme

The pET-28a(+) vector, harboring the sequence coding for the truncated human CBS, was transformed into DE3 bacteria, *i.e.*, HMS174(DE3) or BL-21(DE3), and kanamycin-resistant clones were selected and maintained as stock glycerol at -80°C for further use.

Bacteria from stock glycerol was grown in 5 ml of Luria-Bertani (LB) medium, containing 30 ug/ml kanamycin, overnight at 37°C on a rotational shaker at 275 RPM. The next morning, 1 ml of the overnight culture was added to a 100 ml Terrific Broth (TB) medium containing 30 ug/ml kanamycin and grown overnight as described above, 10 ml of the overnight culture was then added to a 1 liter TB medium containing the following supplements:
- 0.001% of thiamine-HCl pH 8.0
- 0.0025% of pyridoxine-HCl pH 8.0
- 0.3 mM δ-(aminolevulinic acid) (δ-ALA) pH 8.0
- 150 µM ferric chloride
- 30 ug/ml of kanamycin

The 1 liter culture was then grown at 30°C on a rotational shaker at 275 RPM until OD₆₀₀ reached the value of ∼0.6-0.7 and protein expression was induced by addition of 1 mM IPTG. Fermentation was continued for additional 16 hours. Cells were then harvested by a 10 minutes, 6000 RCF centrifugation at 4°C, washed with ice-cold 0.9% NaCl, re-centrifuged as above, and frozen at -80°C.

An aliquot, 4.45 ml of lysis buffer (20 mM NaH₂PO₄, pH=7.2, 40 mM NaCl, 0.1 mM PLP) per 1 gram of pellet, was then added to the cell pellet and the latter was homogenized in a Dounce homogenizer until no cells aggregates were visible. The homogenate was then treated with lysozyme (2 mg/ml final), incubated for 1 hour at 4°C on a rocking platform, sonicated to reduce viscosity, and centrifuged at 53,000 RCF. The supernatant, comprising the soluble fraction was then stored at -80°C,

Expression levels were verified by gel-electrophoresis followed by Coomassie gel staining, and specific activity was determined toy the radioactive activity assay.

### c. CBS Assay

CBS activity was determined by a previously described radioisotope assay using [¹⁴C] serine as the labeled substrate (Kraus, 1987, Methods Enzymol. 143,388-394). Protein concentrations were determined by the Bradford procedure (Bradford, 1976, Anal. Biochem. 72, 248-254) using bovine serum albumin (BSA) as a standard. One unit of activity is defined as the amount of CBS that catalyzes the formation of 1 µmol of cystathionine in 1 h at 37°C.

### d. Denaturing and Native Polyacrylamide Gel Electrophoresis and Western blotting

Western blot analysis of CBS samples under both denaturing and native conditions was performed as described previously (Majtan et al., 2010 J Biol Chem. 2010; 285(21):15866-73).

### e. Determination of plasma metabolites

Stable-Isotope-Dilution Liquid Chromatography-Mass Spectrometry method was used to measure levels of sulfur amino acid metabolism compounds in mouse plasma, substantially as disclosed in Allen et al., 1993, Serum betaine N, N-dimethylglycine and N-methylglycine levels in individuals with cobalamin and folate deficiency and related inborn errors of metabolism, Metabolism 42: 1448-1460.

### Example 2

### Plasma retention time in vivo is enhanced following rhCBSΔC PEGylation.

In order to assess the retention time and activity of rhCBSΔC in blood circulation, experiments were performed where C57BL/6J mice were injected with 5 mg/kg body weight of the rhCBSΔC *via* intra-peritoneal (IP), intra-vascular (IV) or sub-cutaneous (SQ) routes. To avoid excessive bleeding, two experimental groups (denoted 1 and 2) of five mice each were used for each injection route (total n=30). For each injection route, blood was collected from group 1 at 0, 1, 8 and 24 hours post injection and from group 2 at 1, 4, 10, and 48 hours post injection. Animals were bled by a single-use lancet for submandibular bleeding and blood was collected into Capiject T-MLHG lithium heparin (12.5 IU) tubes with gel (Terumo Medical Corporation, NJ, USA). Tubes were then centrifuged at 1200G for 10 min, followed by collection of plasma to 1.5 ml tubes and storage at -80°C.

Plasma was analyzed for CBS activity using the radiometric activity assay set forth in Example 1. CBS enzyme activity for the injected enzyme at the indicated time-points is shown in Figure la. For the IP and IV routes, the peak activity was recorded 1h post injection, whereas for the SQ route, activity peaked at 4h post injection, due to the slower release from the SQ compartment to the circulation. Interestingly, 8-10 hours post injection, the activity was comparable for all injection routes with almost no activity at 24-48 hours post injection.

To monitor whether rhCBSΔC clearance from circulation may contribute to the rapid loss of activity *in vivo* as shown above, plasma proteins from two representative mice from each group described above were resolved by electrophoresis, transferred onto a PVDF membrane and reacted with an anti-human CBS antibody to follow CBS clearance from circulation. As shown in Figure 1b, gradual clearance of the rhCBSAC from circulation occurs as a function of time post injection, with no enzyme detected as early as 24h post injection. Accordingly, clearance of the rhCBSΔC contributes to the observed rapid loss of activity *in vivo.*

In order to prolong the retention time of rhCBSΔC, the enzyme was modified by polyethylene glycol (PEG) molecules (PEGylated) with ME-020MA or GL4-400MA PEGs. Activated PEG derivatives were purchased from the NOF Corporation (Tokyo, Japan). PEGylation was carried out according to the manufacturer's instructions. For example, coupling of PEG maleimide derivatives to the SH groups of CBS (5 mg/ml) was carried out in a 100 mM phosphate buffer pH=6 overnight at 4°C. Molar ratio of PEG molecules to the CBS protein was 10:1 or 5:1, depending on the activated PEG derivative.

To evaluate the activity of the PEGylated rhCBSΔC, C57BL/6J mice were injected with 5 mg/kg body weight of ME020MA- or GL4-400MA-PEGylated rhCBSΔC or with non-PEGylated rhCBSΔC via SQ route. Each treatment arm consisted of two groups of live mice (total 30 mice) and bled as described in above. Plasma was analyzed for CBS activity using the radiometric activity assay set forth in Example 1. Significant activity was detected for the two forms of the PEGylated enzyme 24h and 48h post injection, with GL4-400MA PEGylated rhCBSΔC activity peaking at 24h, as shown in Figure 1c. This was in sharp contrast to non-PEGylated rhCBSAC, demonstrating low or no activity at these same time points. These results indicated that PEGylation of the rhCBSΔC enzyme was effective in prolonging its activity both *in vitro* and *in vivo.*

### Example 3

### Repeated injection regimen with PEGylated, but not with non-PEGylated rhCBSΔC, exhibited accumulation of CBS activity in vivo.

Rapid clearance rate of a protein from the circulation may be bypassed by increasing the number of injections to maintain higher plasma concentrations. Accordingly, a repeated injection regimen was tested with both non-PEGylated and PEGylated rhCBSΔC. C57BL/6J mice were injected at 0, 24, 48 hours (arrows in Figure 2) with 5 mg/kg body weight of non-PEGylated rhCBSΔC (n=5) or with GL4-400MA rhCBSΔC (n=5) and bled at the indicated time points. Plasma was analyzed for CBS activity using the radiometric activity assay set forth in Example 1. As shown in Figure 2, repeated injections of non-PEGylated enzyme did not lead to accumulated enzyme activity in circulation, resulting in nearly no activity 24h after each injection. In contrast, the activity of the PEGylated enzyme peaked after two injections, and reached a plateau.

### Example 4

### Single injection of the PEGylated rhCBSΔC reduced homocysteine and increased cystathionine in plasma.

The preceding Examples were carried out in wild-type mice and were focused on characterization of the clearance and activity of the injected rhCBSΔC, and comparison between the PEGylated and the non-PEGylated enzyme. Once established that PEGylation prolongs circulation time, a varied repertoire of PEGylated CBS enzyme molecules were first tested in wild-type mice, and were then evaluated in a mouse model of homocystinuria.

A repertoire of rhCBSΔC enzymes modified with various PEG molecules were tested in wild-type mice in order to determine the best PEGylation strategy. Twenty seven C57BL/6J mice were divided into nine experimental groups (n=3). Each experimental group was injected via SQ route with 5 mg/kg body weight of rhCBSΔC PEGylated with the PEG molecule indicated in Figure 3, or injected with non-PEGylated enzyme. Blood samples were drawn at the time points indicated in Figure 3 and the PEGylated rhCBSΔC activity determined using the radiometric activity assay set forth in Example 1. Data is presented in Figure 3A as a histogram with standard deviation (STD), and as a scatter plot. Generally, PEGylation with PEGs of higher molecular weights (GL2800MA, 80 kDa; ME-400MA and GL2-400MA, 40 kDa; and ME200-MA0B, 20 kDa) lead to greater exposure than that observed after PEGylation with molecules that were less than 20 kDa in size, and PEGylation using chemistry that targeted cysteine residues (denoted by "MA" to indicate the use of a maleimide reactive group) generally lead to greater exposure than that observed following PEGylation utilizing other chemistries.

Before proceeding with studies on HO mice, daily fluctuations in homocysteine, cystathionine, cysteine and methionine in these mice were assayed to determine the diurnal fluctuation of these amino acids in these animals. Accordingly, blood from six HO mice was drawn at the time points indicated in Figure 3B throughout a 24 hour cycle and plasma metabolites levels were determined for each indicated time point. As shown in Figure 3B, levels of cystathionine and cysteine were largely constant throughout a 24 hour cycle. Homocysteine and methionine levels tended to decrease from 7:00 in the morning to late morning - early afternoon hours. This was consistent for animals which eat at night and lack the capacity for homocysteine metabolism via the trans-sulfuration pathway. Accordingly, all injections and bleedings were performed at 15:00 (3PM), when the Hey levels were the lowest, to determine whether treatment resulted in a further decrease of Hey and other metabolites.

The molecular weight and specific activity (S.A.) of PEGylated rhCBSΔC are central properties for characterization. Following PEGylation with ME-400MA (lane I, Figure 3C), GL4-400MA (lane 2) or ME-200MA0B (lane 3), the products were resolved by electrophoresis on a 12% SDS-PAGE gel and compared with the non-PEGylated enzyme (lane 4). The resulting gel was stained with Coomassie blue. The S.A. (U/mg) for each of the PEGylated and non-PEGylated rhCBSΔC is indicated in the table accompanying Figure 3C. As shown, PEGylation had no significant effect on S.A. values, and the enzyme remained as active as it was prior to PEGylation. According to the apparent molecular weights, PEGylation of rhCBSΔC under the conditions used in this experiment produced di- and tri-PEGylated rhCBSΔC.

The overall goal of the experiments described herein was to assess pharmacodynamic parameters of administered PEGylated rhCBSAC. Special emphasis was placed on lowering of plasma homocysteine and increasing of plasma cystathionine in an animal model of CBS deficient homocystinuria. HO mice fed with a normal murine diet were selected as the model system for testing this type of enzyme replacement therapy (ERT). HO mice were injected once at time 0 with rhCBSΔC that was PEGylated with the PEG molecules (GL4-400MA, ME-400MA, ME-200MA0B), and bled at times 0 (prior to injection), 24, 48 and 72 hours. Levels of plasma homocysteine (Figure 3D) and cystathionine (Figure 3E) for each of the groups (n=5-6) are indicated. As shown in Figure 3D. homocysteine levels decreased significantly for each PEGylated form used, and cystathionine levels (Figure 3E) increased about 6 to 7-fold compared to time zero. Thus, administration of a PEGylated rhCBSΔC was found to significantly and positively impact homocysteine and cystathionine levels *in vivo.*

### Example 5

### Repeated injection of the PEGylated rhCBSΔC significantly impacted homocysteine and cystathionine plasma levels, and restored normal cysteine levels.

A repeated injection regimen with a washout period, comparing non-PEGylated and PEGylated rhCBSΔC for the ability to reduce and maintain low levels of homocysteine as well as to increase cystathionine and cysteine levels was conducted as follows. Six HO mice were injected (arrows, Figure 4) with the rhCBSΔC PEGylated with GL4-400MA PEG, on days 0, 1, 2, 1 and 4, followed by a 10 day washout and then injected again on days 14, 15, 16, 17 and 18. Plasma samples were drawn (always prior to injection) at the time points indicated in Figure 4. For comparison, same injection regimen was carried out in five HO mice injected with the non-PEGylated enzyme. The levels of plasma metabolites were determined by Stable-Isotope-Dilution Liquid Chromatography-Mass Spectrometry as described in Example 1e. Homocysteine (results shown in Figure 4a) and cystathionine (Figure 4b) plasma concentrations for each mouse are indicated. Average values for homocysteine and cystathionine for each experimental group are presented as well (in Figure 4c). As shown in Figures 4a-4c, average homocysteine concentrations decreased in 48 hours from 182 µM to 38 µM and stayed low for the entire week. Cystathionine reached a concentration of 42 µM at 48 hours, from the initial 4.7 µM at time 0, and remained high during the first week. During the washout period, metabolites returned to their initial values. Subsequent CBS injections led again to a sharp drop in homocysteine levels and a rise in cystathionine levels. Thereafter, discontinuation of treatment again resulted in return of these parameters to untreated levels.

The effects of PEGylated rhCBSΔC on plasma homocysteine levels as compared to non-PEGylated rhCBSΔC was also determined, as presented in Figure 4d as percentage of time 0. Figure 4d illustrates that, in contrast to PEGylated rhCBSΔC, the non-PEGylated enzyme had no apparent effect on homocysteine concentration when blood samples were 24 hours or more after injection. This is in agreement with the results shown in Examples 2 and 3 showing that the non-PEGylated enzyme is rapidly cleared from circulation with no significant activity present 24 hours and 48 hours post injection.

The effects of PEGylated rhCBSΔC on plasma cysteine levels as compared to the non-PEGylated rhCBSΔC was also determined using the same experimental methods. As shown in Figure 4E, cysteine levels normalized (doubled as compared to time 0) during the injection period of the PEGylated enzyme, while no change in cysteine levels was observed using the non-PEGylated enzyme.

These results showed that PEGylated rhCBSΔC enzyme administered SQ to HO mice, significantly and positively impacted homocysteine and cystathionine concentrations, and at the same time restored cysteine levels to their normal values. The latter experimental result is an indication that the intracellular transsulfuration pathway was activated following administration of rhCBSΔC.

### Example 6

### Variability in the degree of aggregation of human truncated CBS preparations (rhCBSΔC).

Native PAGE gels (4-15% *i.e*., without the addition of denaturants such as sodium dodecyl sulfate) were run with different batches (112, 13, 7, 27 and 28) of rhCBSΔC. The results portrayed in Figure 5A illustrate the different preparations have varying ratios of tetramers (T) and dimers (D), and higher forms of aggregated CBS. In-gel CBS activity (Figure 5B) was determined in the native polyacrylamide gel (by activity staining) by performing electrophoresis on the protein samples at 4°C. Then, the gel was submerged in an activity staining solution (see Table A) and was incubated at 37°C for about 15 minutes. After about 15 minutes dark gray bands (∼45kDa dimers of the I-413 CBS species) appeared, depending on the amount loaded. Tetramers became visible after much longer time; up to one hour or overnight at room temperature. Analyzing native gels with this in-gel activity method, as exemplified in Figure 5B, confirms that bands of tetramers (T), dimers (D), and other higher aggregated forms of rhCBSΔC appearing in batches 1 12 and 28 in Fig5A were CBS related.

**Table A. Activity staining solutions.**

| *Activity staining procedure:* | | | |
|---|---|---|---|
| **Component** | **Final Conc. [mM]** | **Stock solution** | **Amount of stock soln.** |
| Tris-HCl, pH 8.0 | 100 | 1M | 10ml |
| L-cysteine | 20 | 1M | 2ml |
| 2-mercaptoethanol | 50 | 14.2M | 352.1ul |
| PLP | 0.1 | 10mM | 1ml |
| Lead nitrate | 0.2 | 100mM | 200ul |
| **Total volume** | | | **100ml** |
| Stop solution: | 7 % acetic acid | Prepared as 7% | |

SDS PAGE analysis of batches 28 and 112 and their PEGylated products showed that PEGylation with ME-200MA0B of rhCBSΔC batches showed different ratios between the two PEGylated bands (P) that are formed following PEGylation (see Fig. 5C). Thus, means to reduce aggregation and increase reproducibility were sought.

### Example 7

### Human C15S mutant CBS only forms dimers.

The cysteine, at the position 15 of rhCBSΔC, is uncoupled, so it was considered a possible factor that lead to aggregate formation. Therefore, a new recombinant plasmid was prepared for producing the rhCBSΔC mutant protein, herein referred to as CiSS. The same strategy that was used in Example It to make rhCBSΔC was used to generate the C15S mutant CBS enzyme (T to A nucleotide mutation) by using primers:
**C1** 5'- TGGGTCCGACGGGTAGCCCGCAC - 3' (SEQ ID NO: 11) and
**C2** 5' - GTGCGGGCTACCCGTCGGACCCA - 3' (SEQ ID NO: 12).

The entire nucleotide sequence (SEQ ID NO: 14) of the C15S mutant CBS truncate enzyme, was verified by nucleotide sequencing. The same methods described in Example 1 for the recombinant human CBS truncate were used for expression and isolation of the C15S mutant CBS enzyme.

Native PAGE of isolated C15S CBS enzymes demonstrated that C15S CBS existed only as dimers (see Figure 6A), unlike the results obtained for recombinant human truncated CBS (rhCBSΔC) that formed dimers (D) and tetramers (T), as well as higher oligomeric forms. Multiple batches (51, 60 and 73) of the C15S mutant CBS and recombinant human double truncate (batch RC-2-76) demonstrated that they both form only dimers as opposed to the recombinant human truncated CBS (rhCBSΔC) (see Figure 6B). PEGylation of C15S with ME-200MA0B gave rise to uniform and reproducible bands, with similar ratios between the PEGylated bands. A denaturing SDS PAGE showed that C15S produced a reproducible PEGylation pattern which was similar to recombinant human double truncate (batch RC-2-76) and both were different from the pattern obtained using recombinant human truncated CBS (rhCBSΔC) (Figure 6C). This was also comparable to Fig 5C, showing non-reproducible PEGylation patterns with rhCBSΔC.

HPLC size exclusion chromatography of the CBS preparations was performed. Both recombinant human truncated CBS (rhCBSAC) (Figure 7A) and C15S mutant (Figure 7B) were separated on Yana SEC-3000, 300 × 7.8 mm size exclusion column (Phenomenex, CA, USA). The column was calibrated and operated in 100 mM sodium phosphate pH=6.8, at a flow rate of 1 ml/minute at room temperature. Figure 7 shows size exclusion chromatography (SEC) analyses of rhCBSΔC (Figure 7A) and C15S mutant (Figure 7B). These analyses provide additional evidence that the C15S mutant CBS existed solely as a dimer, as it gave a reproducible single peak from five different batches (see Fig7B). For comparison, note the six different patterns of rhCBSAC in Fig7A and the single peaks of the C15S mutant CBS in Fig7B.

### Example 8

### Continuous administration of PEGylated C15S over 20 days in two HO mice resulted in a decrease in plasma homocysteine.

The ALZET^{®} osmotic pumps permitted steady controlled delivery of the C15S enzyme for a prolonged period of time. The C15S CBS enzyme was administrated using the ALZET^{®} pumps (Micro-osmotic ALZET^{®} Pump, Model 1002, Lot 10268-12) to continuously deliver the C15S CBS enzyme to mice instead of repeated subcutaneous injections. In these experiments, mean pumping rate was 0.21 ul/hr; mean fill volume was 106.6 ul; the pumps were loaded with approximately 106 ul of the PEGylated C15S CBS enzyme at a concentration of 26.2 ug/ul. Continuous administration of C15S CBS over 20 days in two HO mice resulted in an initial significant and subsequently sustained decrease in plasma homocysteine (see Figure 8).

For pain relief pre- and post-operatively, animals received TYLENOL^{®} (2mg/ml) in water bottles *ad libitum* for 48 hours before and after surgery for implantation of ALZET^{®} pump. In addition the animals received Carprofen 5 mg/kg, subcutaneously, every 24 hours, for 48 hours postoperatively.

For animal anesthesia and surgery, isoflurane was administered by inhalation. Mice were induced with 5% Isoflurane and maintained at 2-3%. While mice were anesthetized, they each received a medical grade ALZET^{®} osmotic pump implanted under sterile conditions in a Biological Safety Cabinet. Pumps were implanted subcutaneously in the loose skin area on the ventral side of the neck. Prior to implanting the pump, the mice were shaved and the skin area was prepped with BETADINE^{®}. Pump implantation required a small surgical incision (5mm) made with sterile scissors. The incision was closed with a wound clip. Clips were removed after 1 week when the skin had fully healed. Just prior to pump implantation and at the times indicated in Fig. 8, blood samples were taken for measuring homocysteine levels. The results indicate that this mode of administration lowers homocysteine levels in a manner similar achieved by daily rhCBSΔC administration. A similar experiment carried out using non-PEGylated C15S mutant CBS resulted in no apparent decrease in Hey plasma levels (results not shown).

### Example 9

### Comparison of three PEGylated truncated human CBS enzymes.

Figure 9 is a bar graph showing the results of experiments wherein HO mice were injected with a single dose (7.5 mg/kg) at time 0 with rhCBSΔC, C15S mutant (C15S) and the double truncated construct (double truncate) that had been PEGylated with ME-200MA0B. The mice were bled at times 0 (prior to injection), 24, 48 and 72 hours post injection. Levels of plasma homocysteine for each of the groups (n=5-6) are indicated. Results show that all three enzyme forms lead to a comparable decrease in Hey plasma concentrations.

**Table 2: CBS Sequences**

| **Molecule** | **Sequence** |
|---|---|
| **Human CBS nucleic acid sequence (full length)** | ATGCCGTCAGAAACCCCGCAGGCAGAAGTGGGTCCGACGGGTTGCCCGCACCGTAGCGGT CCGCATTCTGCAAAAGGCAGTCTGGAAAAAGGTTCCCCGGAAGATAAAGAAGCCAAAGAA CCGCTGTGGATTCGTCCGGACGCACCGTCACGCTGTACCTGGCAGCTGGGTCGTCCGGCA AGCGAATCTCCGCATCACCATACGGCTCCGGCGAAAAGTCCGAAAATTCTGCCGGATATC CTGAAGAAAATTGGTGACACCCCGATGGTTCGTATCAACAAAATCGGCAAAAAATTCGGT CTGAAATGCGAACTGCTGGCTAAATGTGAATTTTTCAATGCGGGCGGTTCCGTGAAAGAT CGTATCTCACTGCGCATGATTGAAGATGCTGAACGCGACGGCACCCTGAAACCGGGTGAT ACGATTATCGAACCGACCTCTGGCAACACGGGTATCGGTCTGGCACTGGCGGCGGCAGTC CGTGGTTATCGCTGCATTATCGTGATGCCGGAAAAAATGAGCTCTGAAAAAGTTGATGTC CTGCGTGCTCTGGGCGCGGAAATTGTTCGTACCCCGACGAATGCCCGCTTCGACAGTCCG GAATCCCATGTGGGTGTTGCATGGCGCCTGAAAAACGAAATCCCGAATTCGCACATTCTG GATCAGTATCGTAACGCTAGCAATCCGCTGGCGCATTACGATACCACGGCCGACGAAATC CTGCAGCAATGTGATGGCAAACTGGACATGCTGGTCGCTTCTGTGGGTACCGGCGGTACC ATTACGGGCATCGCGCGTAAACTGAAAGAAAAATGCCCGGGCTGTCGCATTATCGGTGTG GATCCGGAAGGCAGTATTCTGGCGGAACCGGAAGAACTGAACCAGACCGAACAAACCACG TATGAAGTTGAAGGCATCGGTTACGATTTTATTCCGACCGTCCTGGATCGCACGGTGGTT GACAAATGGTTCAAAAGCAATGACGAAGAAGCCTTTACCTTCGCACGTATGCTGATCGCT CAGGAAGGTCTGCTGTGCGGTGGTTCAGCAGGTTCGACGGTCGCAGTGGCAGTTAAAGCT GCGCAGGAACTGCAAGAAGGTCAACGTTGTGTCGTGATTCTGCCGGATTCTGTTCGCAAC TACATGACCAAATTTCTGAGTGACCGTTGGATGCTGCAAAAAGGCTTCCTGAAAGAAGAA GATCTGACCGAGAAAAAACCGTGGTGGTGGCACCTGCGCGTGCAGGAACTGGGTCTGTCC GCACCGCTGACCGTTCTGCCGACCATCACGTGCGGCCATACGATTGAAATCCTGCGTGAA AAAGGTTTTGATCAGGCCCCGGTTGTCGACGAAGCAGGCGTGATTCTGGGTATGGTTACC CTGGGTAACATGCTGAGTTCCCTGCTGGCGGGCAAAGTGCAACCGAGCGATCAGGTTGGT AAAGTCATCTACAAACAATTCAAACAGATTCGTCTGACCGATACGCTGGGCCGCCTGTCG CACATCCTGGAAATGGACCATTTCGCGCTGGTTGTGCACGAACAGATTCAATACCATAGC ACCGGCAAATCATCGCAGCGCCAAATGGTCTTTGGTGTCGTGACGGCCATTGATCTGCTG AATTTCGTGGCCGCACAAGAACGTGACCAGAAATAA |
| **SEQ ID NO: 01** | |
| **Human CBS polypeptide Sequence (full length enzyme)** | |
| **SEQ ID NO: 02** | |
| **Polypeptide Sequence of rhCBSΔC (recombinant human carboxy-truncated CBS)** | MPSETPQAEVGPTGCPHRSGPHSAKGSLEKGSPEDKEAKEPLWIRPDAPSRCTWQLGRPA SESPHHHTAPAKSPKILPDILKKIGDTPMVRINKIGKKFGLKCELLAKCEFFNAGGSVKD RISLRMIEDAERDGTLKPGDTIIEPTSGNTGIGLALAAAVRGYRCIIVMPEKMSSEKVDV LRALGAEIVRTPTNARFDSPESHVGVAWRLKNEIPNSHILDQYRNASNPLAHYDTTADEI LQQCDGKLDMLVASVGTGGTITGIARKLKEKCPGCRIIGVDPEGSILAEPEELNQTEQTT YEVEGIGYDFIPTVLDRTVVDKWFKSNDEEAFTFARMLIAQEGLLCGGSAGSTVAVAVKA AQELQEGQRCVVILPDSVRNYMTKFLSDRWMLQKGFLKEEDLTEKKPWWWHLR |
| **SEQ ID NO: 03** | |
| **Nucleic acid sequence of rhCBSΔC** | ATGCCGTCAGAAACCCCGCAGGCAGAAGTGGGTCCGACGGGTTGCCCGCACCGTAGCGGT CCGCATTCTGCAAAAGGCAGTCTGGAAAAAGGTTCCCCGGAAGATAAAGAAGCCAAAGAA CCGCTGTGGATTCGTCCGGACGCACCGTCACGCTGTACCTGGCAGCTGGGTCGTCCGGCA AGCGAATCTCCGCATCACCATACGGCTCCGGCGAAAAGTCCGAAAATTCTGCCGGATATC CTGAAGAAAATTGGTGACACCCCGATGGTTCGTATCAACAAAATCGGCAAAAAATTCGGT CTGAAATGCGAACTGCTGGCTAAATGTGAATTTTTCAATGCGGGCGGTTCCGTGAAAGAT CGTATCTCACTGCGCATGATTGAAGATGCTGAACGCGACGGCACCCTGAAACCGGGTGAT ACGATTATCGAACCGACCTCTGGCAACACGGGTATCGGTCTGGCACTGGCGGCGGCAGTC CGTGGTTATCGCTGCATTATCGTGATGCCGGAAAAAATGAGCTCTGAAAAAGTTGATGTC CTGCGTGCTCTGGGCGCGGAAATTGTTCGTACCCCGACGAATGCCCGCTTCGACAGTCCG |
| **SEQ ID NO: 04** | |
| | GAATCCCATGTGGGTGTTGCATGGCGCCTGAAAAACGAAATCCCGAATTCGCACATTCTG GATCAGTATCGTAACGCTAGCAATCCGCTGGCGCATTACGATACCACGGCCGACGAAATC CTGCAGCAATGTGATGGCAAACTGGACATGCTGGTCGCTTCTGTGGGTACCGGCGGTACC ATTACGGGCATCGCGCGTAAACTGAAAGAAAAATGCCCGGGCTGTCGCATTATCGGTGTG GATCCGGAAGGCAGTATTCTGGCGGAACCGGAAGAACTGAACCAGACCGAACAAACCACG TATGAAGTTGAAGGCATCGGTTACGATTTTATTCCGACCGTCCTGGATCGCACGGTGGTT GACAAATGGTTCAAAAGCAATGACGAAGAAGCCTTTACCTTCGCACGTATGCTGATCGCT CAGGAAGGTCTGCTGTGCGGTGGTTCAGCAGGTTCGACGGTCGCAGTGGCAGTTAAAGCT GCGCAGGAACTGCAAGAAGGTCAACGTTGTGTCGTGATTCTGCCGGATTCTGTTCGCAAC TACATGACCAAATTTCTGAGTGACCGTTGGATGCTGCAAAAAGGCTTCCTGAAAGAAGAA GATCTGACCGAGAAAAAACCGTGGTGGTGGCACCTGCGCTAA |
| **Truncated human C15S mutant CBS polypeptide Sequence** | MPSETPQAEVGPTGSPHRSGPHSAKGSLEKGSPEDKEAKEPLWIRPDAPSRCTWQLGRPA SESPHHHTAPAKSPKILPDILKKIGDTPMVRINKIGKKFGLKCELLAKCEFFNAGGSVKD RISLRMIEDAERDGTLKPGDTIIEPTSGNTGIGLALAAAVRGYRCIIVMPEKMSSEKVDV LRALGAEIVRTPTNARFDSPESHVGVAWRLKNEIPNSHILDQYRNASNPLAHYDTTADEI LQQCDGKLDMLVASVGTGGTITGIARKLKEKCPGCRIIGVDPEGSILAEPEELNQTEQTT YEVEGIGYDFIPTVLDRTVVDKWFKSNDEEAFTFARMLIAQEGLLCGGSAGSTVAVAVKA AQELQEGQRCVVILPDSVRNYMTKFLSDRWMLQKGFLKEEDLTEKKPWWWHLR |
| **SEQ ID NO: 13** | |
| **Human C15S mutant CBS Nucleotide Sequence (C15S with C-terminal truncate)** | ATGCCGTCAGAAACCCCGCAGGCAGAAGTGGGTCCGACGGGTAGCCCGCACCGTAGCGGT CCGCATTCTGCAAAAGGCAGTCTGGAAAAAGGTTCCCCGGAAGATAAAGAAGCCAAAGAA CCGCTGTGGATTCGTCCGGACGCACCGTCACGCTGTACCTGGCAGCTGGGTCGTCCGGCA AGCGAATCTCCGCATCACCATACGGCTCCGGCGAAAAGTCCGAAAATTCTGCCGGATATC CTGAAGAAAATTGGTGACACCCCGATGGTTCGTATCAACAAAATCGGCAAAAAATTCGGT CTGAAATGCGAACTGCTGGCTAAATGTGAATTTTTCAATGCGGGCGGTTCCGTGAAAGAT CGTATCTCACTGCGCATGATTGAAGATGCTGAACGCGACGGCACCCTGAAACCGGGTGAT ACGATTATCGAACCGACCTCTGGCAACACGGGTATCGGTCTGGCACTGGCGGCGGCAGTC CGTGGTTATCGCTGCATTATCGTGATGCCGGAAAAAATGAGCTCTGAAAAAGTTGATGTC CTGCGTGCTCTGGGCGCGGAAATTGTTCGTACCCCGACGAATGCCCGCTTCGACAGTCCG GAATCCCATGTGGGTGTTGCATGGCGCCTGAAAAACGAAATCCCGAATTCGCACATTCTG GATCAGTATCGTAACGCTAGCAATCCGCTGGCGCATTACGATACCACGGCCGACGAAATC CTGCAGCAATGTGATGGCAAACTGGACATGCTGGTCGCTTCTGTGGGTACCGGCGGTACC ATTACGGGCATCGCGCGTAAACTGAAAGAAAAATGCCCGGGCTGTCGCATTATCGGTGTG GATCCGGAAGGCAGTATTCTGGCGGAACCGGAAGAACTGAACCAGACCGAACAAACCACG TATGAAGTTGAAGGCATCGGTTACGATTTTATTCCGACCGTCCTGGATCGCACGGTGGTT GACAAATGGTTCAAAAGCAATGACGAAGAAGCCTTTACCTTCGCACGTATGCTGATCGCT CAGGAAGGTCTGCTGTGCGGTGGTTCAGCAGGTTCGACGGTCGCAGTGGCAGTTAAAGCT GCGCAGGAACTGCAAGAAGGTCAACGTTGTGTCGTGATTCTGCCGGATTCTGTTCGCAAC TACATGACCAAATTTCTGAGTGACCGTTGGATGCTGCAAAAAGGCTTCCTGAAAGAAGAA GATCTGACCGAGAAAAAACCGTGGTGGTGGCACCTGCGCTAA |
| **SEQ ID NO: 14** | |
| **Human CBS nucleic acid sequence (full length with T to A mutation to codon 15)** | ATGCCGTCAGAAACCCCGCAGGCAGAAGTGGGTCCGACGGGTAGCCCGCACCGTAGCGGT CCGCATTCTGCAAAAGGCAGTCTGGAAAAAGGTTCCCCGGAAGATAAAGAAGCCAAAGAA CCGCTGTGGATTCGTCCGGACGCACCGTCACGCTGTACCTGGCAGCTGGGTCGTCCGGCA AGCGAATCTCCGCATCACCATACGGCTCCGGCGAAAAGTCCGAAAATTCTGCCGGATATC CTGAAGAAAATTGGTGACACCCCGATGGTTCGTATCAACAAAATCGGCAAAAAATTCGGT CTGAAATGCGAACTGCTGGCTAAATGTGAATTTTTCAATGCGGGCGGTTCCGTGAAAGAT CGTATCTCACTGCGCATGATTGAAGATGCTGAACGCGACGGCACCCTGAAACCGGGTGAT ACGATTATCGAACCGACCTCTGGCAACACGGGTATCGGTCTGGCACTGGCGGCGGCAGTC CGTGGTTATCGCTGCATTATCGTGATGCCGGAAAAAATGAGCTCTGAAAAAGTTGATGTC CTGCGTGCTCTGGGCGCGGAAATTGTTCGTACCCCGACGAATGCCCGCTTCGACAGTCCG GAATCCCATGTGGGTGTTGCATGGCGCCTGAAAAACGAAATCCCGAATTCGCACATTCTG GATCAGTATCGTAACGCTAGCAATCCGCTGGCGCATTACGATACCACGGCCGACGAAATC CTGCAGCAATGTGATGGCAAACTGGACATGCTGGTCGCTTCTGTGGGTACCGGCGGTACC ATTACGGGCATCGCGCGTAAACTGAAAGAAAAATGCCCGGGCTGTCGCATTATCGGTGTG GATCCGGAAGGCAGTATTCTGGCGGAACCGGAAGAACTGAACCAGACCGAACAAACCACG TATGAAGTTGAAGGCATCGGTTACGATTTTATTCCGACCGTCCTGGATCGCACGGTGGTT GACAAATGGTTCAAAAGCAATGACGAAGAAGCCTTTACCTTCGCACGTATGCTGATCGCT |
| **SEQ ID NO: 15** | |
| | CAGGAAGGTCTGCTGTGCGGTGGTTCAGCAGGTTCGACGGTCGCAGTGGCAGTTAAAGCT GCGCAGGAACTGCAAGAAGGTCAACGTTGTGTCGTGATTCTGCCGGATTCTGTTCGCAAC TACATGACCAAATTTCTGAGTGACCGTTGGATGCTGCAAAAAGGCTTCCTGAAAGAAGAA GATCTGACCGAGAAAAAACCGTGGTGGTGGCACCTGCGCGTGCAGGAACTGGGTCTGTCC GCACCGCTGACCGTTCTGCCGACCATCACGTGCGGCCATACGATTGAAATCCTGCGTGAA AAAGGTTTTGATCAGGCCCCGGTTGTCGACGAAGCAGGCGTGATTCTGGGTATGGTTACC CTGGGTAACATGCTGAGTTCCCTGCTGGCGGGCAAAGTGCAACCGAGCGATCAGGTTGGT AAAGTCATCTACAAACAATTCAAACAGATTCGTCTGACCGATACGCTGGGCCGCCTGTCG CACATCCTGGAAATGGACCATTTCGCGCTGGTTGTGCACGAACAGATTCAATACCATAGC ACCGGCAAATCATCGCAGCGCCAAATGGTCTTTGGTGTCGTGACGGCCATTGATCTGCTG AATTTCGTGGCCGCACAAGAACGTGACCAGAAATAA |
| **Human** CBS **polypeptide Sequence (full length enzyme with C15S mutation)** | MPSETPQAEVGPTGSPHRSGPHSAKGSLEKGSPEDKEAKEPLWIRPDAPSRCTWQLGRPA SESPHHHTAPAKSPKILPDILKKIGDTPMVRINKIGKKFGLKCELLAKCEFFNAGGSVKD RISLRMIEDAERDGTLKPGDTIIEPTSGNTGIGLALAAAVRGYRCIIVMPEKMSSEKVDV LRALGAEIVRTPTNARFDSPESHVGVAWRLKNEIPNSHILDQYRNASNPLAHYDTTADEI LQQCDGKLDMLVASVGTGGTITGIARKLKEKCPGCRIIGVDPEGSILAEPEELNQTEQTT YEVEGIGYDFIPTVLDRTVVDKWFKSNDEEAFTFARMLIAQEGLLCGGSAGSTVAVAVKA AQELQEGQRCVVILPDSVRNYMTKFLSDRWMLQKGFLKEEDLTEKKPWWWHLRVQELGLS APLTVLPTITCGHTIEILREKGFDQAPVVDEAGVILGMVTLGNMLSSLLAGKVQPSDQVG KVIYKQFKQIRLTDTLGRLSHILEMDHFALVVHEQIQYHSTGKSSQRQMVFGVVTAIDLL NFVAAQERDQK |
| **SEQ ID NO: 16** | |

Having described the invention in detail and by reference to specific embodiments thereof, it will be apparent that modifications and variations are possible without departing from the scope of the invention defined in the appended claims. More specifically, although some aspects of the present invention are identified herein as particularly advantageous, it is contemplated that the present invention is not necessarily limited to these particular aspects of the invention.
The present disclosure also includes the following items.
1. An isolated cystatbionine-β-synthase (CBS) polypeptide comprising SEQ ID NO: 02 or homologue, variant or mutant thereof, wherein the isolated CBS polypeptide is chemically modified and comprises a truncation of the full-length human CBS protein at the amino terminus, carboxyl terminus or both ammo and carboxyl termini.
2. The isolated CBS polypeptide of item 1, wherein the isolated CBS polypeptide is a genetically engineered truncation that is a deletion of C-terminal residues 383-551, 397-551. 414-551, 442-551, 489-551, 497-551, 524-551, 534-551 or 544-551.
3. The isolated CBS polypeptide of item 2, wherein the isolated CBS polypeptide comprises a deletion of C-terminal residues 414-551.
4. The isolated CBS polypeptide of any of items 1-3, wherein the isolated CBS polypeptide is a genetically engineered truncation selected from a deletion of N-terminal residues 2-39 or 1-70.
5. The isolated CBS polypeptide of item 1, wherein the isolated CBS polypeptide comprises a change of a cysteine at amino acid position 15 to serine.
6. The isolated CBS polypeptide of item 5, wherein the isolated CBS polypeptide comprising a change of a cysteine at amino acid position 15 to serine is a genetically engineered truncation selected from a deletion of C-terminal residues 383-551, 397-551, 414-551, 442-551, 489-551, 497-551, 524-551, 534-551or 544-551.
7. The isolated CBS polypeptide of item 6, wherein the isolated CBS polypeptide comprising a change of a cysteine at amino acid position 15 to serine also comprises a deletion of C-terminal residues 414-551 (SEQ ID NO: 13).
8. An isolated CBS polypeptide comprising SEQ ID NO: 02 or homologue thereof wherein the isolated CBS polypeptide comprises a change of a cysteine at amino acid position 15 to serine, and wherein the isolated CBS polypeptide is chemically modified.
9. The isolated CBS polypeptide of any of items 1-8, wherein the CBS polypeptide is covalently linked to one or a plurality of polyethylene glycol molecules.
10. The isolated CBS polypeptide of item 9, wherein the polyethylene glycol molecules are selected from unbranched and branched polyethylene glycol.
11. The isolated CBS polypeptide of item 10, wherein the polyethylene glycol molecules have a molecular weight that equals of exceeds 2000 Daltons.
12. The isolated CBS polypeptide of item 11, wherein the polyethylene glycol molecules have a molecular weight in the range of 5 - 80 kD.
13. The isolated CBS polypeptide of item 11, wherein the polyethylene glycol molecules preferably have a molecular weight in the range of 10 - 40 kD.
14. The isolated CBS polypeptide of item 11, wherein the polyethylene glycol molecule is ME-200MA0B, ME-020MA, ME-400MA. GL2-400MA, GL4-400MA, GL2-800MA, ME-050GS, ME-200GS, ME-200AL or MEPA-20T.
15. The isolated CBS polypeptide of any of items 8-14, wherein the polyethylene glycol molecules are attached to CBS via an attachment group selected from the group consisting of: NHS, amine, and aldehyde consisting of a monoaldehyde, a monoester of a monoacid, a monoamine, a monothiol, a monodisulfide, a monobromophenyl carbonate, a monochlorophenyl carbonate, a monofluorophenyl carbonate, a mononitrophenyl carbonate, a monocarbonylmidazole, a monohydrazide, a monoiodoacetamide, a monomaleimide, a monoorthopyridyl disulfide, a monooxime, a monophenyl glyoxal, a monothiazolidine-2-thione, a monothioester, a monotriazine and a monovinylsulfone.
16. The isolated CBS polypeptide of any of items 1-15, wherein the CBS polypeptide is active in the absence of S-Adenosylmethionine (AdoMet).
17. A pharmaceutical composition comprising the isolated CBS polypeptide of any of items 1-16 comprising a therapeutically effective amount of the polypeptide, homologue, variant or mutant thereof and a pharmaceutically acceptable carrier, diluent or excipient.
18. The pharmaceutical composition of items 1-17 that retains 70% or more of the initial activity of the administered composition after 24 hours.
19. The pharmaceutical composition of item 17 that retains 70% or more of the initial activity of the administered composition after 24 hours.
20. The pharmaceutical composition of any of items 17-19. wherein the CBS polypeptide is covalently linked to one or a plurality of polyethylene glycol molecules.
21. A method for reducing homocysteine in an individual in need thereof comprising administration of the isolated CBS polypeptide of any of items 1-20.
22. The method of item 21, wherein the isolated CBS polypeptide is administered by an implanted osmotic pump, intravenous injection, subcutaneous injection, or intraperitoneal injection.
23. The method of item 21, wherein the isolated CBS polypeptide is administered by subcutaneous injection.
24. The method of item 21, wherein the isolated CBS polypeptide is administered by an implanted osmotic pump.
25. The method of any of items 21-24, wherein the isolated CBS polypeptide comprises an amount of the isolated CBS polypeptide wherein 0.1 mg/kg - 20 mg/kg is administered,
26. A method for elevating cystathionine or cysteine, in an individual in need thereof comprising administration of the isolated CBS polypeptide of any of items 1-20.
27. The method of item 26, further comprising the administration of one or more of an anti-coagulant, a statin, or a relaxed protein restricted diet, anethole dithiolethione or betaine.
28. The method of item 26, wherein the isolated CBS polypeptide is administered by an implanted osmotic pump, intravenous injection, subcutaneous injection, or intraperitoneal injection.
29. The method of item 26, wherein the isolated CBS poly-peptide is administered by intravenous injection,
30. The method of item 26, wherein the isolated CBS polypeptide is administered by osmotic pump,
31. The method of any of items 26-30, wherein the isolated CBS polypeptide comprises an amount of the isolated CBS polypeptide wherein up to 20 mg/kg is administered to an individual in need thereof
32. A method for treating or ameliorating a disease, disorder, or condition, associated with elevated homocysteine, methionine, S-adenosylmethionine and S-adenosylliomocysteine and low cystathionine and cysteine levels comprising administering to an individual in need thereof a pharmaceutically effective amount of the isolated CBS polypeptide of any of items 1-20.
33. The method of item 32, wherein the disease, disorder, or condition associated with elevated homocysteine is mental retardation, osteoporosis, ectopia lentis, kyphoscoliosis, stroke, myocardial infarction, or pulmonary embolism.
34. The method of item 32, wherein the isolated CBS polypeptide comprises an amount of the isolated CBS polypeptide wherein up to 20 mg/kg is administered by parenteral injection.
35. The method of item 32, wherein the isolated CBS polypeptide comprises an amount of the isolated CBS polypeptide wherein up to 20 mg/kg is administered by an implanted osmotic pump.
36. The method of item 32, further comprising administration of anethole dithiolethione or betaine.
37. The method of item 32, wherein betaine administration occurs twice daily,
38. A method for preventing a disease, disorder, or condition associated with elevated homocysteine, comprising administering to an individual in need thereof a pharmaceutically effective amount of the isolated CBS polypeptide of any of items 1-20.
39. The method of item 38, wherein the disease, disorder, or condition associated with elevated homocysteine is mental retardation, osteoporosis, ectopia lends, kyphoscoliosis, stroke, myocardial infarction, or pulmonary embolism.
40. The method of item 38, wherein the isolated CBS polypeptide comprises an amount of the isolated CBS polypeptide wherein up to 20 mg/kg is administered to an individual in need thereof.
41. The method of item 38, further comprising administration of anethole dithiolethione or betaine.
42. The method of item 38, wherein betaine administration occurs twice daily.
43. The use of the isolated CBS polypeptide of any of items 1-20, for the manufacture of a medicament for treating or ameliorating a disease, disorder, or condition associated with elevated homocysteine,
44. The use of item 43 wherein the disease, disorder, or condition associated with elevated homocysteine is mental retardation, osteoporosis, ectopia lentis, kyphoscohosis, stroke, myocardial infarction, or pulmonary embolism.
45. The use of item 43, wherein the medicament further comprises anethole dithiolethione or betaine.
46. The use of item 43 further comprising restriction to a relaxed protein restricted diet.

## Claims

1. An isolated polypeptide dimer comprising two monomeric human truncated cystathionine β-synthase (htCBS) mutant polypeptide subunits, wherein each monomeric htCBS mutant polypeptide subunit independently comprises:
an amino acid sequence sharing an amino acid sequence identity of at least 50%, at least 60%, at least 70%, at least 80%, at least 90%, at least 95%, or at least 99% compared with SEQ ID NO:2, wherein the amino acid sequence of the htCBS mutant polypeptide further comprises
a carboyxyl-terminal truncation, wherein the truncation comprises residues 383-551, 397-551, 414-551, 442-551, 489-551, 497-551, 524-551, 534-551, or 544-551 of SEQ ID NO: 2; and
a mutation of a cysteine to a serine at amino acid position 15 compared to a cystathionine β-synthase (CBS) protein comprising the amino acid sequence of SEQ ID NO: 2;
wherein each htCBS mutant polypeptide subunit is independently covalently linked to at least one polyethylene glycol (PEG) molecule, wherein the at least one PEG molecule is an NHS ester-activated PEG molecule or has a molecular weight greater than or equal to 20 kDa.

2. The isolated polypeptide dimer of claim 1, wherein each monomeric htCBS mutant polypeptide subunit independently comprises an amino acid sequence sharing an amino acid sequence identity of at least 90%, more preferably at least 95%, or more preferably at least 99% compared with SEQ ID NO: 2.

3. The isolated polypeptide dimer of claim 1 or 2, wherein the truncation of each monomeric htCBS mutant polypeptide subunit comprises residues 414-551 of SEQ ID NO: 2.

4. The isolated polypeptide dimer of any one of claims 1-3, wherein each monomeric htCBS mutant polypeptide subunit is independently covalently linked to a plurality of PEG molecules, wherein each PEG molecule is an NHS ester-activated PEG molecule or has a molecular weight greater than or equal to 20 kDa.

5. The isolated polypeptide dimer of any one of claims 1-4, wherein each PEG molecule covalently linked to each htCBS mutant polypeptide subunit is independently an unbranched PEG molecule or a branched PEG molecule.

6. The isolated polypeptide dimer of any one of claims 1-5, wherein each PEG molecule covalently linked to each htCBS mutant polypeptide subunit is independently ME-200MA0B, ME-400MA, GL2-400MA, GL4-400MA, GL2-800MA, ME-200GS, ME-200AL or MEPA-20T.

7. The isolated polypeptide dimer of cany one of claims 1-6, wherein:
each monomeric htCBS mutant polypeptide subunit independently comprises an amino acid sequence sharing an amino acid sequence identity of at least 99% compared with SEQ ID NO: 2;
the truncation of each monomeric htCBS mutant polypeptide subunit comprises residues 414-551 of SEQ ID NO: 2; and
each htCBS mutant polypeptide subunit is independently covalently linked to a plurality of NHS ester-activated unbranched PEG molecules, each having a molecular weight greater than or equal to 20 kDa.

8. The isolated polypeptide dimer of any one of claims 1-7, wherein the nucleic acid encoding each htCBS mutant polypeptide subunit is optimized for expression in an *Escherichia coli* (*E. coli*) based expression system.

9. The isolated polypeptide dimer of any one of claims 1-8, wherein the polypeptide dimer is purified from other protein components such that the polypeptide dimer comprises at least about 80% weight/weight of the total protein in a given composition, and more preferably, at least about 85%, and more preferably at least about 90%, and more preferably at least about 91 %, and more preferably at least about 92%, and more preferably at least about 93%, and more preferably at least about 94%, and more preferably at least about 95%, and more preferably at least about 96%, and more preferably at least about 97%, and more preferably at least about 98%, and more preferably at least about 99%, weight/weight of the total protein in a given composition.

10. The isolated polypeptide dimer of any one of claims 1-9, wherein the polypeptide dimer is non-heme binding and remains catalytically active.

11. The isolated polypeptide dimer of any one of claims 1-10, wherein the amino acid sequence of each htCBS mutant polypeptide subunit comprises no more than two amino acid residues at the N-terminus that are not a residue of the naturally occurring human cystathionine β-synthase amino acid sequence at that position.

12. A pharmaceutical composition comprising:
a therapeutically effective amount of the isolated polypeptide dimer of any one of claims 1-11; and
a pharmaceutically acceptable carrier, diluent, or excipient.

13. The pharmaceutical composition of claim 12, for use in the treatment or amelioration of homocystinuria or homocysteine remethylation disorder, wherein the isolated polypeptide dimer is to be administered to an individual in need thereof.

14. The pharmaceutical composition for use of claim 13, wherein the pharmaceutical composition retains 70% or more of the initial activity of the administered composition after 24 hours.

15. The pharmaceutical composition for use of claim 13 or 14, wherein an amount of the isolated polypeptide dimer up to 20 mg/kg, more preferable between 0.1 to 7.5 mg/kg, or more preferably between 0.1 to 5 mg/kg is to be administered to the individual.

16. The pharmaceutical composition for use of any one of claims 13-15,
wherein the homocystinuria or homocysteine remethylation disorder is mental retardation, osteoporosis, ectopia lentis, kyphoscoliosis, stroke, myocardial infarction, or pulmonary embolism.

17. The pharmaceutical composition for use of any one of claims 13-16,
wherein the individual is also administered one or more of an anti-coagulant, a statin, a relaxed protein restricted diet, anethole dithiolethione, or betaine.

18. The pharmaceutical composition for use of claim 17, wherein betaine administration occurs twice daily.

19. The pharmaceutical composition for use of claim 17, wherein the individual is on a relaxed protein restricted diet.

20. The pharmaceutical composition for use of any one of claims 13-19,
wherein the isolated polypeptide dimer is formulated for administration to the individual by an implanted osmotic pump, intravenous injection, subcutaneous injection, or intraperitoneal injection.
